# EUROPEAN PATENT APPLICATION

(11) **EP 1 681 018 A1**
(43) Date of publication of application: **19.07.2006**
(21) Application number: 04792127.5
(22) Date of filing: 07.10.2004
(51) Int. Cl.: A61B 5/08

(54) **SLEEP ASPIRATION STATE MEASUREMENT DEVICE**

(30) Priority: 07.10.2003 JP 2003348141; 05.11.2003 JP 2003375528; 12.12.2003 JP 2003414745; 12.12.2003 JP 2003414746
(71) Applicant: Olympus Corporation, Tokyo 151-0072 (JP)
(72) Inventor: XIE, Tianyu, 1970821 (JP); ISHIHARA, Yasushige, 1920023 (JP); MIURA, Naoki, 1920045 (JP); TOKUDA, Kazunari, 1930834 (JP)
(74) Representative: von Hellfeld, Axel
(86) International application number: PCT/JP2004/014829
(87) International publication number: WO 2005/034750

(57) **Abstract**

(A device) Attached to the nostrils and/or the mouth of a test subject, comprises a respiratory sensor that detects the air pressure changes due to breathing in and breathing out air from the nostrils or the mouth during respiration, and a respiratory information analyzing means that analyzes respiratory information from the information of the air pressure changes detected by the respiratory sensor.

## Description

### TECHNICAL FIELD

The present invention relates to a device for measuring respiratory condition during sleep that measures the respiratory condition of a test subject during sleep.
Priority is claimed on Japanese patent application No. 2003-375528 filed on November 5, 2003, Japanese patent application No. 2003-414746 filed on December 12, 2003, Japanese patent application No. 2003-414745 filed on December 12, 2003, and Japanese patent application No. 2003-348141 filed on October 7, 2003, the contents of which are incorporated herein by reference.

### BACKGROUND ART

In recent years, devices for measuring respiratory condition during sleep are being used to measure the respiratory condition of test subjects during sleep to diagnose apnea syndrome and so on.
Since the past, devices for measuring the respiratory condition of test subjects by attaching naso-oral respiratory sensors provided with thermistors for detecting temperature changes in respiratory air at the entrance of the nostrils and in the center of the mouth of the test subject, are well known among these devices (for example, refer to reference patent 1: Japanese unexamined patent application No. 2000-312669).

However, according to the aforementioned configuration, the thermistor was affected considerably by changes in temperature, such as room temperature, with the passage of time, and it became difficult to measure the respiratory condition of the test subject with good accuracy.

The present invention has been made in consideration of the aforementioned circumstances, and it is an object of the present invention to provide a device for measuring the respiratory condition during sleep that can measure with high accuracy the respiratory condition of a test subject during sleep without being affected by changes in temperature, such as room temperature.

Various kinds of respiratory condition measuring devices for examining the respiratory condition of test subjects are being proposed now, and are attracting attention as effective means for examining the state of respiratory processes, such as respiratory failure and apneic condition during sleep. Also, devices for measuring the respiratory condition of this kind can acquire various kinds of bio-information of a test subject and examine the respiratory condition based on such bio-information. For instance, devices for measuring temperature, humidity, and air pressure of breath during respiration, devices for measuring the amount or concentration of carbon dioxide included in breath during respiration, or devices for measuring respiratory sounds and so on, are well known. The bio-information measuring device that can be used for examining the respiratory condition during sleep is well known (for instance, see reference patent 1) as one of the devices for measuring respiratory condition.

The bio-information measuring device mentioned above comprises an oxygen saturation finger sensor attached to a finger for measuring the oxygen saturation, pulse rate, and so on, a flow sensor with a nasal breath temperature detecting unit and an oral breath temperature detecting unit for detecting changes in temperature of respiratory air after detecting nasal or oral breath, a microphone attached to the throat for detecting tracheal sounds and snoring sounds, and so on. The average value of oxygen saturation, the average value of pulse rate, and apneic frequency per unit time, and so on, are analyzed based on each of these detected results.
Consequently, according to the above-mentioned bio-information measuring device, the apneic frequency and so on during sleep can be easily grasped, and is thus considered to be an effective means for examining the state of process of the apneic condition during sleep.

However, in the bio-information measurement device mentioned in reference patent 1 above, flow sensors need to be attached below the nose using adhesive tape and so on, so that the nasal breath temperature detecting unit is positioned at the entrance of the nostrils, and the oral breath temperature detecting unit is positioned at the center of the mouth. For this reason, the test subject has a strong feeling of being constrained. Also, there were inconveniences, such as these devices were likely to be taken off when the test subject rolled over or moved, and they were susceptible to the effects of body motion and the surrounding environment.

The present invention takes into consideration such circumstances, and its object is to offer a device for measuring the respiratory condition that can examine the respiratory condition when the effect of body motion is reduced, and the feeling of constraint given to the test subject is also reduced.

Various kinds of devices for measuring respiratory condition for examining the respiratory condition of test subjects are being proposed now, and are attracting attention as effective means for examining the state of process, such as respiratory failure and the apneic condition during sleep. Also, such kinds of devices for measuring the respiratory condition can acquire various kinds of bio-information of a test subject and examine the respiratory condition based on such bio-information. For instance, devices that measure temperature, humidity, and air pressure during respiration, or those that measure the amount or concentration of carbon dioxide included during respiration, or those that measure respiratory sound, are well known. The bio-information measuring device that can be used for examining the respiratory condition during sleep is well known (for instance, see reference patent 1) as one such device for measuring the respiratory condition.

The above-mentioned bio-information device includes items such as oxygen saturation finger sensor, flow sensor, and microphone. Here, the oxygen saturation finger sensor is attached to the tip of the finger and it measures oxygen saturation, pulse rate, and so on. Flow sensors detect nasal or oral breaths and also detect changes in temperature of the respiratory air. Also, the microphone is attached to the throat and it detects tracheal sound or snoring sound. In this way, bio-information measuring devices can analyze the average value of oxygen saturation, the average value of pulse rate, the apneic frequency per unit time, and so on, based on the various detected results.
Consequently, according to the above-mentioned bio-information measuring device, the apneic frequency and so on during sleep can be easily grasped, and it is thus considered to be an effective means for examining the state of process of the apneic condition during sleep.

According to the bio-information measuring device mentioned in reference patent 1 above, the initial symptoms and the state of process of the apneic condition during sleep could be grasped. However, for medical treatment, examination using large-scale equipment, such as MRI equipment at medical institutions such as hospitals, is necessary to detect the position of obstruction in the respiratory tract. After detecting the position of obstruction, appropriate medical treatment needs to be received depending on the condition of the obstruction. That is, to detect the position of obstruction in the respiratory tract, a medical institution and the like, needs to be visited and examination needs to be received, which require time and effort.

The present invention takes such circumstances into consideration. Its object is to offer a device for measuring the respiratory condition that can examine the respiratory condition and easily detect the position of obstruction in the respiratory tract.

It is well known that when apneic condition occurs during sleep, the oxygen concentration in the arterial blood reduces, and sleep becomes lighter. Such sleep disorders may lead to cardiovascular diseases and drowsiness; therefore, patients with such anxieties should be examined for abnormal respiration during sleep.
Examination devices used for such purposes include flow sensors for detecting respiration attached under the nose of a test subject using adhesive tape and so on. This flow sensor is connected to the body of the device by cable, and the body of the device is attached to the test subject's wrist (for instance, refer to reference patent 1). Such kinds of examination devices are provided with chest belts for detecting changes, finger sensors for detecting oxygen saturation, and so on, in addition to flow sensors, and all data including detected results of flow sensors are sent to the main body of the device through the connected cable.
Also, processing equipment for analyzing the detected results may be installed at locations distant from the test subject. In such cases, detected results are transmitted by using wired communication or radio communication in the processing equipment (for instance, refer to reference patent 2: Japanese unexamined test subject application number 2003-126064).

If the equipment changes in the test subject are suppressed by the cable; therefore, it is preferable not to use wired connection with the processing equipment. If data is sent or received using radio communication, the feeling of constraint in the test subject can be reduced, but radio communication has the issue that its power consumption is high compared to wired communication.
The present invention has been made in consideration of the above issues, and its object is to provide a device for measuring the respiratory condition during sleep that does not suppress changes in the test subject. It also has as its object to reduce the power consumption of the device for measuring the respiratory condition during sleep.

### DISCLOSURE OF INVENTION

The present invention offers the means mentioned below for resolving the aforementioned issues.
The first aspect of the present invention comprises a detecting unit that detects wave motion signals in a body cavity; and a respiratory information analyzing unit that analyzes the respiratory information from the information in the wave motion signals detected by the detecting unit.
The second aspect of the present invention comprises a device for measuring the respiratory condition during sleep related to the first aspect of the invention wherein, the wave motion signals are variable signals of respiratory air pressure of a test subject, the detecting unit is attached to the nostrils and/or the mouth of the test subject, and the detecting unit comprises a respiratory sensor that detects changes in air pressure with the respiration near the nostrils or the mouth.

According to the device for measuring the respiratory condition during sleep related to the aspects of the inventions mentioned above, when a test subject attaches the device to the nostrils and/or the mouth before going to sleep, and the test subject breathes during sleep so that air is breathed in and out from the nostrils or the mouth, the changes in air pressure that occur due to breathing in and breathing out are detected by the respiratory sensor. The respiratory condition of the test subject is analyzed by the respiratory information analyzing means, based on the information on air pressure changes from the respiratory sensor.

The third aspect of the present invention comprises a device for measuring the respiratory condition during sleep related to the second aspect of the present invention wherein, the respiratory sensor detects the air pressure strength simultaneously with the detection of air pressure changes.
According to the device for measuring the respiratory condition during sleep related to this aspect of the present invention, the air pressure strength is detected simultaneously with the air pressure changes by the respiratory sensor.

The fourth aspect of the present invention comprises a device for measuring the respiratory condition during sleep related to the second aspect of the present invention wherein, the respiratory sensor is a pressure sensor.
According to the device for measuring the respiratory condition during sleep related to this aspect of the present invention, the air pressure changes, or the air pressure changes and the air pressure strength, are detected by the pressure sensor.

The fifth aspect of the present invention comprises a device for measuring the respiratory condition during sleep related to the second aspect of the present invention wherein, the respiratory information analyzing means comprises a first respiratory information analyzing means that analyzes respiratory cycles from the air pressure changes, and a second respiratory information analyzing means that analyzes the respiratory flowrate from the air pressure strength and the air pressure changes.
According to the device for measuring the respiratory condition during sleep related to this aspect of the present invention, the respiratory cycles are analyzed based on the air pressure changes by the first respiratory information analyzing means, and the respiratory flowrate is analyzed based on the air pressure changes and air pressure strength by the second respiratory information analyzing means.

The sixth aspect of the present invention comprises a device for measuring the respiratory condition during sleep related to the second aspect of the present invention comprising a body motion sensor that detects body motion of a test subject during sleep, and a respiratory information correcting unit that corrects the respiratory information based on the body motion information detected by the body motion sensor.
According to the device for measuring the respiratory condition during sleep related to this aspect of the present invention, noise may be generated in the respiratory information because of the body motion that accompanies the rolling over or other motion of the test subject during sleep. However, the body motion of the test subject is detected by the body motion sensor, and based on this body motion information, the noise accompanying the body motion is removed from the respiratory information by the respiratory information correcting means.

The seventh aspect of the present invention comprises a device for measuring the respiratory condition during sleep related to the second aspect of the present invention wherein, the body motion sensor is integrally installed with the respiratory sensor.
According to the device for measuring the respiratory condition during sleep related to this aspect of the present invention, when the respiratory sensor is moved because of the body motion of the test subject, the body motion sensor is also shifted corresponding to this movement.

The eighth aspect of the present invention comprises a device for measuring the respiratory condition during sleep related to the second aspect of the invention wherein, the body motion sensor is an acceleration sensor.
According to the device for measuring the respiratory condition during sleep related to this aspect of the present invention, the body motion due to rolling over and so on of the test subject during sleep can be detected by the acceleration sensor.

The ninth aspect of the present invention comprises a device for measuring the respiratory condition during sleep related to the first aspect of the present invention wherein the wave motion signals are bio-signals corresponding to respiration, the detecting unit comprises an attaching unit for attaching it to an ear and a sensor that detects the bio-signals corresponding to respiration, and the respiratory information analyzing unit comprises a bio-signal measurement unit that measures the respiratory condition based on the bio-signals detected by the sensor.

In the device for measuring the respiratory condition during sleep related to this aspect of the present invention, the detecting unit detects the bio-signals corresponding to respiration in the ear, such as changes in air pressure and air vibrations, and the measurement means measures the respiratory condition of the test subject based on the bio-signals detected by the detecting unit. In this way, by merely attaching the detecting unit it to the ear during sleep, the examination of respiratory condition can be easily performed. Particularly, there is no need to attach the detecting unit near the mouth or the nose as was done conventionally; it can be attached to the ear and the respiratory condition can be examined. Therefore, the feeling of constraint can be reduced, and the probability of the unit coming off during rolling over and so on can be reduced. Also, unlike the conventional measurement of temperature of oral breath and so on, the detecting unit is attached to the ear; therefore, the respiratory condition can be detected correctly since it is unaffected by the surrounding environment, such as temperature of the surroundings and body motion.

The tenth aspect of the present invention comprises a device for measuring the respiratory condition during sleep related to the ninth aspect of the present invention wherein, the sensor is a vibration sensor that detects vibrations in the ear.
In the device for measuring the respiratory condition during sleep related to this aspect of the present invention, the detecting unit detects air vibrations in the ear corresponding to respiration, such as, vibrations in the frequency band of 0 KHz to 50 KHz, using the vibration sensor.

The eleventh aspect of the present invention comprises a device for measuring the respiratory condition during sleep related to the ninth aspect of the present invention wherein, the sensor is an air pressure sensor that detects the air pressure in the ear.
In the device for measuring the respiratory condition during sleep related to this aspect of the present invention, the detecting unit detects the air pressure in the ear corresponding to respiration using the air pressure sensor.

The twelfth aspect of the present invention comprises a device for measuring the respiratory condition during sleep related to the ninth aspect of the present invention wherein, the sensor is a sound sensor that detects sound in the ear.
In the device for measuring the respiratory condition during sleep related to this aspect of the present invention, the detecting unit detects air vibrations in the ear corresponding to respiration, such as, vibrations in the frequency band of 20 Hz to 20 KHz, using the sound sensor.

The thirteenth aspect of the present invention comprises a device for measuring the respiratory condition during sleep related to the ninth aspect of the present invention wherein, the sensor is a compound sensor that detects a plurality of different bio-signals.
In the device for measuring the respiratory condition during sleep related to this aspect of the present invention, the detecting unit detects a plurality of bio-signals such as sound and air pressure in the ear corresponding to respiration using the compound sensor. Consequently, the respiratory condition can be accurately examined.

The fourteenth aspect of the present invention comprises a device for measuring the respiratory condition during sleep related to the ninth aspect of the present invention wherein, the sensor is an acceleration sensor that detects body motion, and the bio-signal measurement unit corrects the respiratory condition based on the detection values detected by the acceleration sensor.
In the device for measuring the respiratory condition during sleep related to this aspect of the present invention, the body motion during sleep of the test subject can be detected by the acceleration sensor. The respiratory condition can be corrected based on the detected body motion; therefore, the respiratory condition can be examined more accurately.

The fifteenth aspect of the present invention comprises a device for measuring the respiratory condition during sleep related to the first aspect of the present invention comprising a transmitter that transmits wave motion signals to a respiratory tract through the nostrils and/or the mouth, the detecting unit further comprising a receiver that receives the wave motion signals reflected from within the respiratory tract, and the respiratory information analyzing unit further comprising a wave motion signal measurement unit that measures the respiratory condition based on the wave motion signals received in the receiver.
The sixteenth aspect of the present invention comprises a device for measuring the respiratory condition during sleep related to the fifteenth aspect of the present invention wherein, the measurement means comprises an analyzing unit that analyzes the respiratory condition based on the measured results, and a detecting unit that detects the position of the obstruction member in the respiratory tract based on the results analyzed by the analyzing unit.

In the device for measuring the respiratory condition during sleep, examination signals such as electromagnetic wave and sound wave signals transmitted toward the respiratory tract by the transmitter from the nose or the nostrils proceed toward the lungs while being repeatedly reflected by the wall in the respiratory tract. Moreover, the examination signals that have reached the lungs are reflected at the lungs, return again to the mouth or the nostrils, and are received by the receiver. Measurements such as reflection times are performed by the measurement means. In this case, if an obstruction member exists in the respiratory tract, the examination signals are reflected by the obstruction member before reaching the lungs. Accordingly, the reflection time in such a condition becomes shorter than the reflection time in the normal condition. The difference in the reflection times is analyzed by the analyzing unit. The detecting unit receives the results analyzed by the analyzing unit. For instance, by calculating the distance from the mouth or the nostrils to the obstruction member from the reflection time, the obstruction member in the respiratory tract can be detected.
In this way, by transmitting examination signals in the respiratory tract, receiving the examination signals reflected from within the respiratory tract, the obstruction member can be detected easily without much time and effort, and at the same time, the respiratory condition can be examined. As a result, subsequent medical treatment and so on, can become smoother.

The seventeenth aspect of the present invention comprises a device for measuring the respiratory condition during sleep related to the fifteenth aspect of the present invention wherein, the transmitter and the receiver constitute the same device.
In the device for measuring the respiratory condition during sleep related to this aspect of the present invention, the transmitter and the receiver can be made to constitute the same device. As a result, the number of parts can be reduced, cost can be reduced, and the device can be made more compact.

The eighteenth aspect of the present invention comprises a device for measuring the respiratory condition during sleep related to the fifteenth aspect of the present invention wherein, the transmitter and the receiver are attached close to the mouth and/or the nostrils.
In the device for measuring the respiratory condition during sleep, the transmitter and the receiver are attached near the mouth or the nose; therefore, examination signals can be transmitted within the respiratory tract and received from the respiratory tract more accurately.

The nineteenth aspect of the present invention comprises a device for measuring the respiratory condition during sleep related to the fifteenth aspect of the present invention comprising an examination signal propagation guide that can propagate the examination signals, is disposed between the transmitter or the receiver and the mouth or the nostrils. The examination signals are transmitted or received through this propagation guide.
In the device for measuring the respiratory condition during sleep related to this aspect of the present invention, the examination signals are transmitted in the respiratory tract correctly through the examination signal propagation guide, and they are received from the respiratory tract correctly. Also, there is no need to attach the transmitter and the receiver near the mouth or the nostrils because the examination signal propagation guide is used. As a result, the feeling of constraint can be reduced.

The twentieth aspect of the present invention comprises a device for measuring the respiratory condition during sleep related to the fifteenth aspect of the present invention wherein, the wave motion signals are electromagnetic wave signals, the transmitter is an electromagnetic wave transmitter that transmits the electromagnetic wave signals, and the receiver is an electromagnetic wave receiver that receives the electromagnetic wave signals.
The device for measuring the respiratory condition during sleep related to this aspect of the present invention can detect the obstruction position in the respiratory tract using electromagnetic waves, and can examine the respiratory condition.

The twenty-first aspect of the present invention comprises a device for measuring the respiratory condition during sleep related to the fifteenth aspect of the present invention wherein, the wave motion signals are sound wave signals, the transmitter is a sound wave transmitter that transmits the sound wave signals, and the receiver is a sound wave receiver that receives the sound wave signals.
The device for measuring the respiratory condition during sleep related to this aspect of the present invention can detect the obstruction position in the respiratory tract using sound waves, and can examine the respiratory condition.

The twenty-second aspect of the present invention comprises a device for measuring the respiratory condition during sleep related to the fifteenth aspect of the present invention wherein, the wave motion signals are pulse-type signals.
The device for measuring the respiratory condition during sleep related to this aspect of the present invention can detect the obstruction position in the respiratory tract using pulse-type signals, and can examine the respiratory condition.

The twenty-third aspect of the present invention comprises a device for measuring the respiratory condition during sleep related to the first aspect of the present invention comprising a transmitting device that transmits signals that are output from the detecting unit, a receiving device that receives signals transmitted from the transmitting unit, a radio communication means for radio communications installed in the transmitting device and the receiving device, and a communication switching means that switches on and off the radio communications performed between the transmitting device and the receiving device.
According to this device for measuring the respiratory condition during sleep, data can be transmitted and received between devices installed on the side of the test subject and the receiving devices located at a distance from the test subject using radio communications. Furthermore, radio communications can be switched on and off with the communication switching means; therefore, the time for performing radio communications can be reduced.

The twenty-fourth aspect of the present invention comprises a device for measuring the respiratory condition during sleep related to the twenty-third aspect of the present invention comprising a fault determination means that determines whether respiration of the test subject is normal or abnormal, based on the signals output by the detecting unit.
According to this device for measuring the respiratory condition during sleep, the fault determination means determines the respiratory condition of the test subject. The determined results of the fault determination means may be used, for instance, to switch radio communications on and off. This fault determination means may be provided in the communication switching means, or may be provided separately from the communication switching means.

The twenty-fifth aspect of the present invention comprises a device for measuring the respiratory condition during sleep related to the twenty-fourth aspect of the present invention comprising a display means that displays the results determining whether the respiration of the test subject is normal or abnormal, or displays signals expressing the respiratory condition of the test subject.
According to this device for measuring the respiratory condition during sleep, the respiratory condition of the test subject can be visually confirmed by this display means. This display means can be integrally installed with the receiving device, or it may be separately installed.

The twenty-sixth aspect of the present invention comprises a device for measuring the respiratory condition during sleep related to the twenty-fourth aspect of the present invention comprising an apneic time measuring unit that measures the duration of the apneic condition.
According to this device for measuring the respiratory condition during sleep, the duration (apneic condition time) of the apneic condition can be used as the determination algorithm for fault determination. When the apneic time exceeds the time set beforehand, respiration is judged as abnormal; if it is less than the time set beforehand, the respiration is judged as normal. The apneic time measuring unit may be installed in the fault determination means, or it may be installed separate from the fault determination means.

The twenty-seventh aspect of the present invention comprises a device for measuring the respiratory condition during sleep related to the twenty-sixth aspect of the present invention wherein the fault determination means is configured such that it transmits signals expressing the respiratory condition of the test subject only when the respiration of the test subject is in an apneic condition for a time longer than a specific time.
According to this device for measuring the respiratory condition during sleep, radio communication is performed only when the apneic condition has been judged; therefore, the overall communication time can be reduced.

The twenty-eighth aspect of the present invention comprises a device for measuring the respiratory condition during sleep related to the twenty-seventh aspect of the present invention wherein, the fault determination means comprises a respiration cycle measuring unit that measures the respiration cycle.
According to this device for measuring the respiratory condition during sleep, the respiration cycle is used as the determination algorithm for fault determination. When there is practically no disturbance in the respiration cycle, normal respiration is judged; when there is disturbance in the respiration cycle, abnormal respiration is judged.

The twenty-ninth aspect of the present invention comprises a device for measuring the respiratory condition during sleep related to the twenty-third aspect of the present invention wherein, the transmitting device transmits signals that express the respiratory condition of the test subject once per respiration cycle of the test subject.
According to this device for measuring the respiratory condition during sleep, signals are transmitted only once for each respiration cycle, therefore, the radio communication time is shortened compared to the time when signals are continuously transmitted during one respiration cycle.

The thirtieth aspect of the present invention comprises a device for measuring the respiratory condition during sleep related to the twenty-third aspect of the present invention wherein, the transmitting device comprises an arm attaching means for attaching to the wrist of the test subject.
According to this device for measuring the respiratory condition during sleep, devices from the detecting device to the transmitting device can be attached to the test subject. Also, the receiving device can be installed at a location distant from the test subject.

The thirty-first aspect of the present invention comprises a device for measuring the respiratory condition during sleep related to the twenty-third aspect of the present invention wherein the detecting device and the transmitting device are integrally installed.
According to this device for measuring the respiratory condition during sleep, devices from the detecting device to the transmitting device can be attached to the test subject. Moreover, cables for connections from the detecting device to the transmitting device are not required.

The thirty-second aspect of the present invention comprises a device for measuring the respiratory condition during sleep related to the twenty-third aspect of the present invention wherein a means for attachment/removal is provided for freely attaching/removing the detecting device and the transmitting device.
According to this device for measuring the respiratory condition during sleep, the transmitting device can be attached/removed with respect to the detecting device. For instance, when the detecting device and the transmitting device are connected by cable, the cable can be freely attached/removed. Also, if the detecting device and the transmitting device are configured as an integral unit, engaging means and so on can be provided.

The thirty-third aspect of the present invention comprises a device for measuring the respiratory condition during sleep related to the twenty-third aspect of the present invention wherein the receiving device comprises a data transmitting means that transmits data obtained from the detecting device to other equipment.
According to the device for measuring the respiratory condition during sleep, analysis, display, and saving of data in other equipment become possible.

The thirty-fourth aspect of the present invention comprises a device for measuring the respiratory condition during sleep related to the first aspect of the present invention comprising a transmitting device that transmits signals output from the detecting unit, a receiving device that receives signals transmitted from the transmitting device, a radio communication means for radio communication provided in the transmitting device and the receiving device, and a feedback control means in the transmitting device and the receiving device that controls the strength of the transmitted signals of the transmitting device to the minimum required limit according to the sensitivity of the received signals of the receiving device.
According to this device for measuring the respiratory condition during sleep, data can be transmitted and received using the devices mounted on the side of the test subject and the radio communication between the receiving devices at a position distant from the test subject.
Moreover, by feedback control of the strength of transmission signals, the output of the transmitting device can be limited to the minimum required limit.

The thirty-fifth aspect of the present invention comprises a device for measuring the respiratory condition during sleep related to the thirty-fourth aspect of the present invention wherein the feedback control means comprises a reception strength measuring means that measures the strength of received signals provided in the receiving device, and a transmission output adjusting means that adjusts the output of transmission signals based on the strength of the received signals transmitted by radio communication from the reception strength measuring means.
This device for measuring the respiratory condition during sleep measures the reception signal strength on the side of the receiving device, and based on this measurement, creates the output control signal of the transmitting device, transmits this control signal to the side of the test subject by radio communication, and controls the output of the transmitting device.

The thirty-sixth aspect of the present invention comprises a device for measuring the respiratory condition during sleep related to the first aspect of the present invention comprising an analyzing means that analyzes the signals obtained in the detecting unit, and an information exchange means for transferring information through recording media to the detecting unit and the analyzing means.
According to this device for measuring the respiratory condition during sleep, data transfer between the devices attached to the test subject and the analyzing means at a position distant from the test subject can be performed using recording media; therefore, wired connections and radio communications are not necessary.

The thirty-seventh aspect of the present invention comprises a device for measuring the respiratory condition during sleep related to the thirty-sixth aspect of the present invention comprising a first attaching means that attaches the recording media to the detecting unit, a data writing means that writes data to the recording media, a second attaching means that attaches the recording media to the analyzing means, and a data reading means that reads data from the recording media.
This device for measuring the respiratory condition during sleep comprises an information exchange means that includes a first attaching means, a data writing means, a second attaching means, and a data reading means.

The thirty-eighth aspect of the present invention comprises a device for measuring the respiratory condition during sleep related to the thirty-sixth aspect of the present invention wherein the analyzing means is a general-purpose computer.
This device for measuring the respiratory condition during sleep includes a configuration wherein the analyzing means is a general-purpose computer installed with specific analyzing algorithms.

### [Effect of the invention]

According to the second aspect of the present invention, air pressure changes due to breathing in and breathing out of air from the nostrils or the mouth are detected by the respiratory sensor. Therefore, the respiratory condition of a test subject can be measured with high accuracy without receiving the effects of changes in temperature, such as room temperature.
According to the third aspect of the present invention, not only air pressure changes but also air pressure strength is detected; therefore the respiratory condition of a test subject can be measured with higher accuracy.
According to the fourth aspect of the present invention, the air pressure changes or the air pressure strength can be accurately detected by pressure sensors.
According to the fifth aspect of the present invention, respiratory cycles are analyzed by the first respiratory information analyzing means, and respiratory flowrate is analyzed by the second respiratory information analyzing means; therefore, the respiratory condition of a test subject can be measured in detail.

According to the sixth aspect of the present invention, noise generated from body motion due to rolling over and so on, of a test subject can be removed by the respiratory information correcting unit; therefore, the respiratory condition of a test subject can be measured with better accuracy.
According to the seventh aspect of the present invention, the body motion sensor is installed integrally with the respiratory sensor; therefore, correct body motion information matching the movement of the respiratory sensor can be detected.
According to the eighth aspect of the present invention, body motion information can be correctly detected by the acceleration sensor.

According to the device for measuring the respiratory condition during sleep related to the ninth to the fourteenth aspects of the present invention, the respiratory condition can be easily examined by attaching the detecting unit during sleep. In this case, respiratory condition can be examined after attaching the detecting unit to the ear; therefore, the feeling of constraint can be reduced, and also the probability of the detecting unit coming off because of body motion and so on, can be reduced. Moreover, respiratory condition can be correctly detected even in a condition in which the effect of the surrounding temperature or body motion is difficult to receive.

According to the device for measuring the respiratory condition during sleep related to the fifteenth to the twenty-second aspects of the present invention, by transmitting the examination signals within the respiratory tract, and by receiving the examination signals reflected from within the respiratory tract, the position of obstruction can be detected easily without incurring time and effort, and the respiratory condition can also be examined at the same time. As a result, the medical treatment and so on, subsequently, can become smoother.

According to the twenty-third aspect of the present invention, by connecting the devices attached on the side of the test subject to receiving devices at positions distant from the test subject using radio communication, the cables between them become unnecessary; thus the feeling of constraint in the patient can be reduced. Furthermore, radio communication can be switched on and off with the communication switching means, and the communication time can be reduced; therefore, the life of the battery in the transmitting device can be prolonged.
According to the twenty-fourth aspect of the present invention, the fault determination means enables the respiratory condition of the test subject to be determined. Particularly, if radio communication is switched on and off according to the results determined by the fault determination means, the communication time can be reduced, and the life of the battery can be prolonged.
According to the twenty-fifth aspect of the present invention, the respiratory condition of the test subject can be confirmed; therefore, appropriate measures can be formulated promptly.
According to the twenty-sixth aspect of the present invention, the respiratory condition can be determined by measuring the apneic time.

According to the twenty-seventh aspect of the present invention, only when it has been determined that the condition is apneic condition, radio communication is performed; therefore, the overall communication time can be reduced. Consequently, the communication time can be reduced, and the life of the battery can be prolonged. According to the twenty-eighth aspect of the present invention, respiratory faults can be determined with the communication switching means by examining the respiratory cycles. According to the twenty-ninth aspect of the present invention, the time required for a one-time communication can be shortened; therefore, the overall communication time can be reduced, and the life of the battery can be prolonged.
According to the thirtieth aspect.of the present invention, the transmitting device can be securely attached to the arm.
Also, the receiving device can be distanced from the test subject; therefore, the feeling of constraint in the test subject can be reduced.
According to the thirty-first aspect of the present invention, the devices from the
detecting device to the transmitting device can be attached to the test subject. Cables are not necessary; therefore, the feeling of constraint in the test subject can be reduced.

According to the thirty-second aspect of the present invention, the detecting device can be configured to freely attach/remove; therefore, the detecting device can be easily replaced and cleaned enabling the detecting device to be maintained in a clean condition at all times.
According to the thirty-third aspect of the present invention, data can be analyzed, displayed, and saved in equipment other than the receiving device; therefore, the receiving device can be made more compact and its cost can be reduced.
According to the thirty-fourth aspect of the present invention, by installing a device that collects data at a location distant from the devices attached to the test subject, radio communication can be performed between the devices; therefore, the feeling of constraint in the patient can be reduced. Moreover, by performing feedback control of strength of signals transmitted by the transmitting device, the output of the transmitting device can be restricted to the minimum required limit; therefore, the life of the battery of the transmitting device can be prolonged.
According to the thirty-fifth aspect of the present invention, the strength of the receiving signals is measured on the side of the receiving device, and the control signal is transmitted to the side of the transmitting device; therefore, wires need not be connected between these devices, and the feeling of constraint in the test subject can be reduced.

According to the thirty-sixth aspect of the present invention, signals detected by the detecting device are recorded in the recording media; therefore, compared to the wired connection between the detecting device and the analyzing means, the feeling of constraint in the patient can be reduced. Furthermore, compared to the case when radio communication is performed, the life of the battery of the detecting device can be prolonged.
According to the thirty-seventh aspect of the present invention, the information exchange means comprises the first attaching means, the data reading means, the second attaching means, and the data writing means; therefore, the feeling of constraint can be reduced by using this simple configuration and the life of the battery can be prolonged.
According to the thirty-eighth aspect of the present invention, a general-purpose computer installed with specific analyzing algorithms is adequate as the analyzing means; therefore, the device configuration becomes simple and the cost can be reduced.

### BRIEF DESCRIPTION OF THE DRAWINGS

FIG. 1 is the schematic configuration drawing showing the first embodiment of the device for measuring the respiratory condition during sleep related to the present invention.
FIG. 2 is an explanatory drawing showing the arithmetic and logic unit of the device for measuring the respiratory condition during sleep in the same embodiment.
FIG. 3 is a block diagram showing the principles of the device for measuring the respiratory condition during sleep in the same embodiment.
FIG. 4 is an explanatory drawing showing the condition of the attached device for measuring the respiratory condition during sleep in the same embodiment.
FIGS. 5A to 5C are graphs that show the respiratory condition of the test subject using the device for measuring the respiratory condition during sleep in the same embodiment. FIG. 5A is an explanatory drawing showing respiratory information from the pressure sensor; FIG. 5B is an explanatory drawing showing body motion information, and FIG 5C is an explanatory drawing showing the respiratory information after the body motion information has been subtracted from the respiratory information.
FIG. 6 is the schematic configuration drawing showing the second embodiment of the device for measuring the respiratory condition during sleep related to the present invention.
FIG. 7 is an explanatory drawing showing the condition of the attached device for measuring the respiratory condition during sleep in the same embodiment.
FIG. 8 is a block diagram showing the principles of the device for measuring the respiratory condition during sleep in the same embodiment.
FIG. 9 is schematic diagram showing the first embodiment of the device for measuring the respiratory condition related to the present invention.
FIG. 10 is a configuration diagram showing an example of the vibration sensor unit of the device for measuring the respiratory condition shown in FIG. 9.
FIG. 11 is a configuration diagram showing the signal detection circuit of the device for measuring the respiratory condition shown in FIG. 9.
FIG. 12 is a waveform showing an example of the electrical signal sent to the signal detection circuit by the vibration sensor unit.
FIG. 13 is a waveform of an example of an electrical signal after it has passed through the low pass filter.
FIG. 14 is the overall configuration drawing that explains the first embodiment of the device for measuring the respiratory condition related to the present invention.
FIG. 15 is the cross section of the sensor unit in the device for measuring the respiratory condition shown in FIG. 14.
FIG. 16 is a waveform of a transmitted electromagnetic wave signal.
FIG. 17 is a waveform of an electromagnetic wave signal received from within the respiratory tract of a test subject in the normal condition.
FIG. 18 shows the status of electromagnetic wave signal transmitted when a constriction exists between the nostrils in the respiratory tract and the mouth.
FIG. 19 is a waveform of an electromagnetic wave signal received in the condition of FIG. 18.
FIG. 20 shows the status of an electromagnetic wave signal transmitted when a constriction exists between the mouth in the respiratory tract and the lungs.
FIG. 21 is a waveform of an electromagnetic wave signal received in the condition of FIG. 20.
FIG. 22 is a cross section drawing showing another example of a sensor unit when a sound wave signal is transmitted.
FIG. 23 shows the condition of an electromagnetic wave signal transmitted in the respiratory tract after an air pipe is disposed between the sensor unit and the respiratory tract.
FIG. 24 is a schematic configuration drawing of the device for measuring the respiratory condition during sleep in the embodiment of the present invention.
FIG. 25 is a block diagram of the device for measuring the respiratory condition during sleep on the side of the test subject.
FIG. 26 is a block diagram of the receiving device in the device for measuring the respiratory condition during sleep.
FIG. 27 is a drawing showing the detected signal.
FIG. 28 is a drawing showing the results of Fourier transformation of the detected signals.
FIG. 29 is a drawing showing an example of the signal transmitted by the transmitting device.
FIG 30 is a drawing showing the configuration of the integrated detecting device.
FIG. 31 is a drawing showing the means for attachment/removal for freely attaching/removing the detecting device.
FIG. 32 is a schematic configuration drawing of the device for measuring the respiratory condition during sleep in the embodiment of the present invention.
FIG. 33 is a block diagram of the device for measuring the respiratory condition during sleep.
FIG. 34 is a schematic configuration drawing of the device for measuring the respiratory condition during sleep in the embodiment of the present invention.
FIG. 35 is an example of the configuration for performing data analysis and data display.
FIG. 36 is a schematic configuration drawing of the device for measuring the respiratory condition during sleep in the embodiment of the present invention.
FIG. 37 is an example of the configuration for performing data analysis and data display.

### BEST MODE FOR CARRYING OUT THE INVENTION

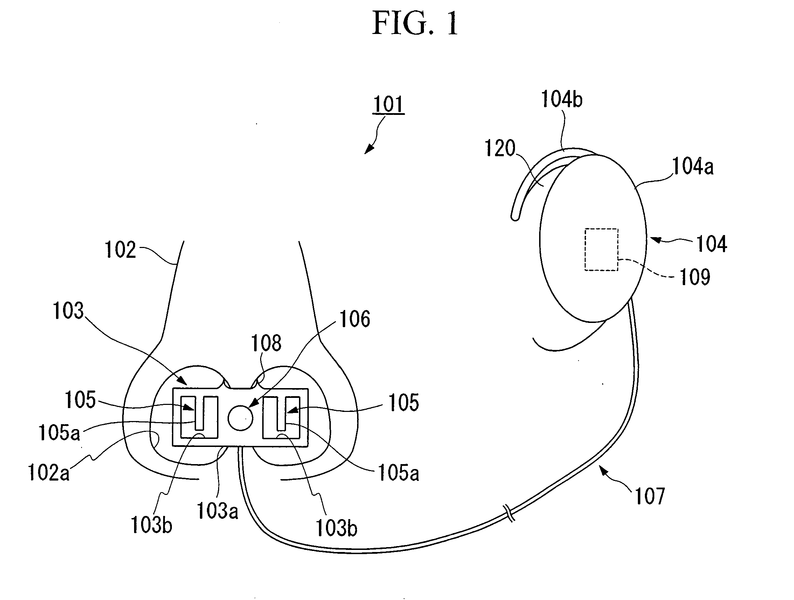

The device for measuring the respiratory condition during sleep of the present invention (hereinafter referred to as "measurement device") is described below referring to the figures.
(First embodiment)
Reference numeral 101 in FIG. 1 shows a device for measuring the respiratory condition during sleep related to the present embodiment, reference number 102 indicates a test subject's nose, and reference numeral 120 indicates a ear.
The device for measuring the respiratory condition during sleep 101 shown in the figure is for measurement of nasal breath, and is connected to a nasal breath sensor (respiratory sensor) 103 and a data processing unit 104 through wire 107. The nasal breath sensor 103 is attachable to the nostrils 102a of a test subject, and has a rectangular shape that bridges the left and right nostrils 102a.
On the other hand, the data processing unit 104 is anchored and attached to the test subject's ear 120.

The nasal breath sensor 103 has a sheet-type substrate member 103a. Rectangular openings 103b are formed near both ends in the longitudinal direction and aligned with the positions of the nostrils of the test subject. These rectangular openings 103b are for allowing the breathing in and breathing out of air by nasal breaths through each of the openings 103b with the nasal breath sensor 103 in the attached to the test subject. Pressure sensors 105 extending in strip shape from the center on one side are provided in these openings 103b. These pressure sensors 105 are piezoelectric elements 105a, the resistance of which varies with the displacement, and these sensors output voltage according to the displacement of the piezoelectric elements 105a.
At the center of the substrate member 103a and between the two openings 103b, an acceleration sensor 106 is provided that acts as a body motion sensor. The acceleration sensor 106 may be for instance, a capacitance-type sensor, provided with fixed-type fixed electrodes not shown in the figures, and movable electrodes that move with the motion of the nasal breath sensor 103. Also, it outputs signals proportional to the width and narrowness, that is, the magnitude of the capacitance, between the fixed electrodes and the movable electrodes.
Furthermore, on the rear face of the nasal breath sensor 103, a clip 108 is fitted to attach the sensor to the nostrils 102a of the test subject.

With such a configuration, let us assume that a test subject has breathed with the nasal breath sensor 103 attached to the test subject's nostrils 102a. Depending on the breathing in and breathing out of air from the nostrils 102a, air flows alternately from within the nostrils 102a to the outside and from the outside toward the inside of the nostrils. The pressure sensors 105 are designed to deform when the flow of air passes through the openings 103b, depending on the strength of the pressure and the direction of pressure of the flow of air. That is, depending on the flow of air, the piezoelectric elements 105a deform toward the inside or toward the outside of the nostrils 102a. Voltage corresponding to the direction of deformation and the deformed amount of the piezoelectric elements 105a is generated from the pressure sensors 105. This voltage is output through the wire 107. The change in the direction of pressure of the flow of air is called air pressure change, while the strength of the pressure of flow of air is called air pressure strength.
Also, when body motion occurs as the test subject rolls over and so on, the movable electrode mentioned above, shifts corresponding to the body motion. As a result, the acceleration sensor 106 generates a signal proportional to the magnitude of the capacitance between the fixed electrodes. This signal is output through the wire 107 as body motion information.

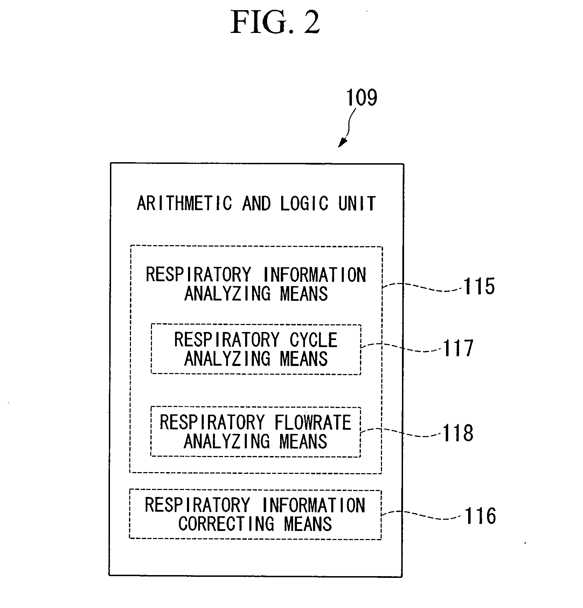

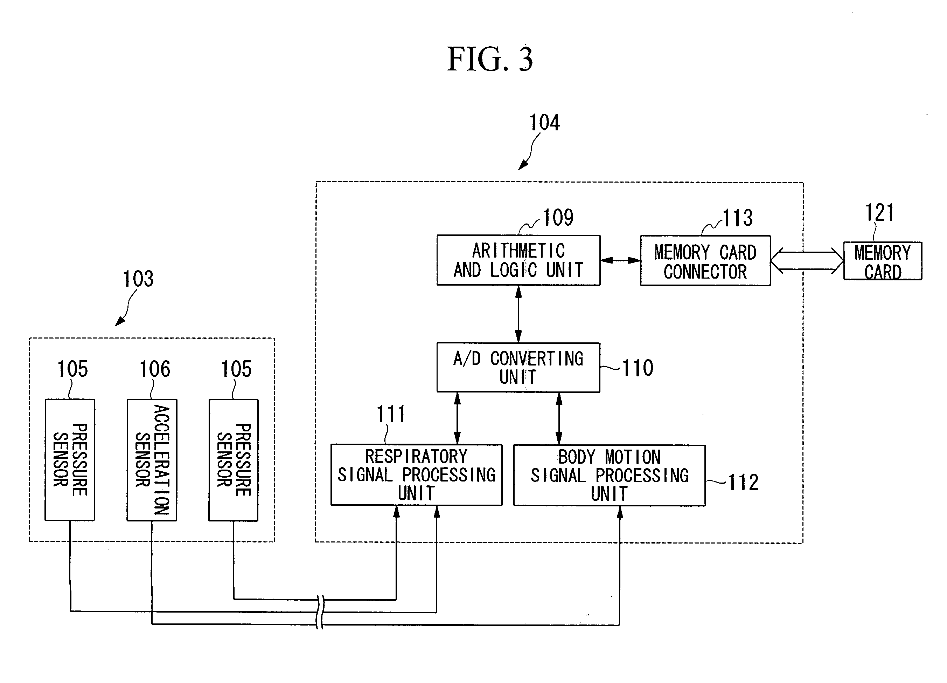

The data processing unit 104 includes a data processing unit body 104a with a built-in arithmetic and logic unit 109 (respiratory information correcting unit) for performing various kinds of arithmetic processing. Furthermore, the data processing unit 104 is provided with a fitting 4b fixed on the outer surface of the data processing unit body 104a. This fitting 4b enables the unit to be attached to the test subject's ear.
As shown in FIG. 2, the arithmetic and logic unit 109 includes a means for analyzing respiratory information 115 (respiratory information analyzing unit) and a means for correcting respiratory information 116, mentioned later. Furthermore, the means for analyzing respiratory information 115 includes a means for respiratory cycle analysis (first respiratory information analyzing means) 117 and a means for respiratory flowrate analysis (second respiratory information analyzing means) 118.
As shown in the block diagram of FIG. 3, the data processing unit body 104a includes a respiratory signal processing unit 111, a body motion signal processing unit 112, an A/D converting unit 110, and a memory card connector 113, in addition to an arithmetic and logic unit 109, which are all built-in. The respiratory signal processing unit 111 and the body motion signal processing unit 112, perform the waveform shaping of the signals output from the pressure sensors 105 and the acceleration sensor 106 respectively. The A/D converting unit 110 converts the signals from the respiratory signal processing unit 111 and the body motion signal processing unit 112 to digital signals. The memory card connector 113 connects the memory card 121 and the arithmetic and logic unit 109. The memory card 121 contains analysis programs.

With this configuration, the signals output from the pressure sensors 105 and the acceleration sensor 106 are subjected to waveform shaping by the respiratory signal processing unit 111 and the body motion signal processing unit 112 respectively. Next, these signals are input to the arithmetic and logic unit 109 through the A/D converting unit 110. Then the specific arithmetic processing is performed by the arithmetic and logic unit 109, and the arithmetically processed results are stored step by step in memory, which is not shown in the figures.

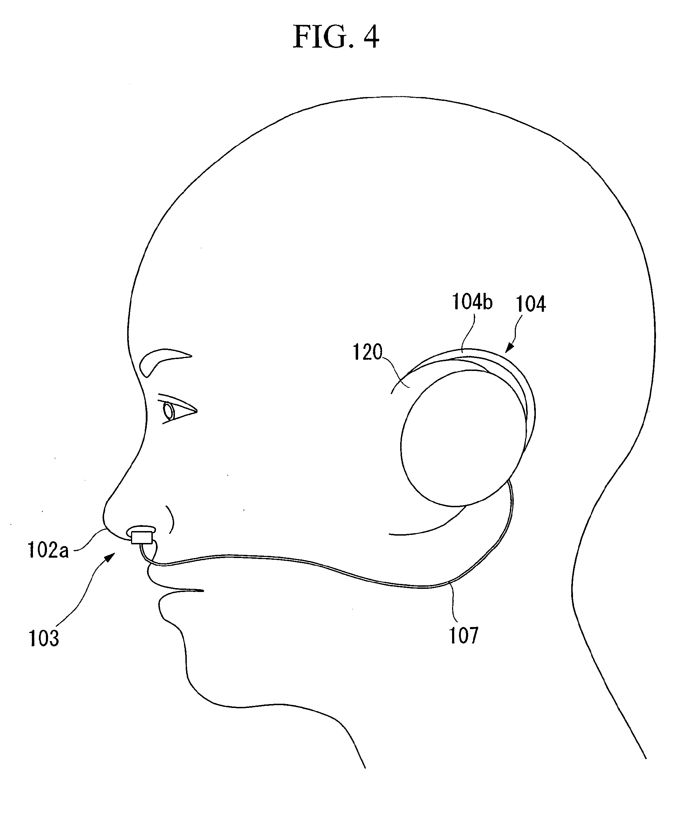

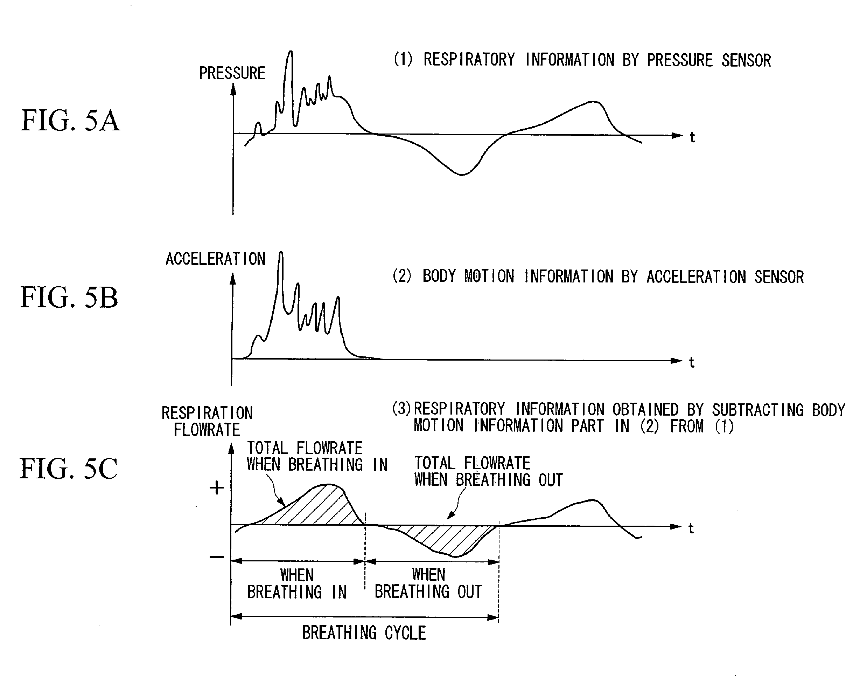

Next, the action of the device for measuring the respiratory condition during sleep 101 is described hereunder.
First, the test subject attaches the device for measuring the respiratory condition during sleep 101 before going to sleep. As shown in FIG. 4, the nasal breath sensor 3 is attached to the nostril 102a, and the data processing unit 104 is attached to the ear 120. In this condition, the test subject lies down on a bed or the like, and sleeps as usual. During sleep, the appropriate respiratory information of the test subject is stored in memory, as mentioned later. After the measurement is completed, the test subject returns the device for measuring the respiratory condition during sleep 101 to the hospital. The doctor performs analysis of the respiratory information mentioned later, in each memory card for the device for measuring the respiratory condition during sleep 101 corresponding to the test subject. As a result, the respiratory condition during sleep is analyzed, as shown in FIG. 5C,.

In the device for measuring the respiratory condition during sleep 101 related to the present embodiment, respiratory information of a test subject is stored in memory and this respiratory information is analyzed, as described hereunder.
That is, when the nasal breath sensor 103 is attached to the test subject's nostrils 102a, and the test subject breathes in through the nostrils 102a, the piezoelectric elements 105a bends inward in the nostrils 102a according to the strength of the air pressure due to the air breathed in. On the other hand, when air is breathed out from the nostrils 102a, the piezoelectric elements 105a bend outward of the nostrils 102a according to the strength of the air pressure due to the air breathed out. Accordingly, as shown in FIG. 5C, in the normal breathing condition, the deformation in the inward and outward directions of the piezoelectric elements 105a mentioned above, occurs alternately and repeatedly at fixed cycles. The specific voltage value is output as respiratory information according to the extent of strength of the air pressure and the extent of air pressure change due to the respiratory air, that is, according to the deformation amount and direction of deformation of the piezoelectric elements 105a by the pressure sensors 105. This output is subjected to waveform shaping by the respiratory signal processing unit 111, converted to digital signals by the A/D converting unit 110, and then input to the arithmetic and logic unit 109. Subsequently, after the specific arithmetic processing by the arithmetic and logic unit 109, the processed results are stored in the step-by-step memory.

When body motion occurs as the test subject rolls over during sleep and so on, noise may be generated as the output from the pressure sensors 105, as shown in FIG. 5A. This noise can be removed in the present embodiment, as described below.
That is, with the body motion, if the nasal breath sensor 103 works together with the test subject, the movable electrode of the acceleration sensor 106 moves, and the width between the movable and fixed electrodes changes.
For this reason, the capacitance between the two electrodes varies, and as shown in FIG. 5B, a signal proportional to the magnitude of this capacitance is output from the acceleration sensor 106 as body motion information. After this output value is subjected to waveform shaping by the body motion signal processing unit 112, it is converted to digital signal by the A/D converting unit 110, and this digital signal is input to the arithmetic and logic unit 109. Subsequently, the above-mentioned body motion information is subtracted by the arithmetic and logic unit 109, the above-mentioned respiratory information is corrected, and the appropriate respiratory information after correction is stored in the step-by-step memory.

Furthermore, the respiratory information is analyzed as described below. Memory card 121 is connected to memory card connector 113, and the analysis program contained in the memory card 121 is run. As a result, the respiratory cycle is calculated based on the respiratory information saved in the above-mentioned memory by the means for respiratory cycle analysis 117. That is, the respiratory cycle is calculated by measuring the specific times of the positive and negative values alternately repeated, taking the breathing in of air as a positive value and breathing out of air as a negative value, as shown in FIG. 5C, according to the changes in the direction of deformation of piezoelectric elements 105a.
The respiratory flow rate is calculated by the means for respiratory flowrate analysis 118. That is, by determining the area of the part surrounded by the curve showing the transition of deformation amount of the piezoelectric elements 105a when breathing in air and the t axis, as shown in FIG. 5C, the total flow rate when air is breathed in can be calculated. Similarly, the total flow rate when air is breathed out is also calculated. As a result, the respiratory cycle and the respiratory flow rate when the test subject is asleep can be calculated, and the respiratory condition of the test subject can be measured in detail.

As mentioned above, the respiratory condition of a test subject can be measured with high accuracy by varying the direction of deformation of the pressure sensors 105 so that the change in air pressure is detected by breathing in/breathing out air from the nostrils 102a unaffected by changes in temperature such as room temperature during measurement by the device for measuring the respiratory condition during sleep 101, related to the present embodiment.
Also, measurements can be made with higher accuracy for detecting the air pressure strength according to the deformation amounts of the pressure sensors 105.
Moreover, measurements at higher accuracy can be made by correcting the respiratory information from the pressure sensors 105 based on the body motion information from the acceleration sensor 106 due to body motion of the test subject.
Furthermore, since the acceleration sensor 106 is integrated in the same unit as the pressure sensors 105, correct body motion information matching the operation of the pressure sensors 105 can be detected.
By using the means for respiratory cycle analysis 117 and the means for respiratory flowrate analysis 118, the respiratory cycle and respiratory flow rate of the test subject during sleep can be calculated; therefore, the respiratory condition of the test subject can be measured in detail.

### (Second embodiment)

Next, the second embodiment of the present invention is described below.

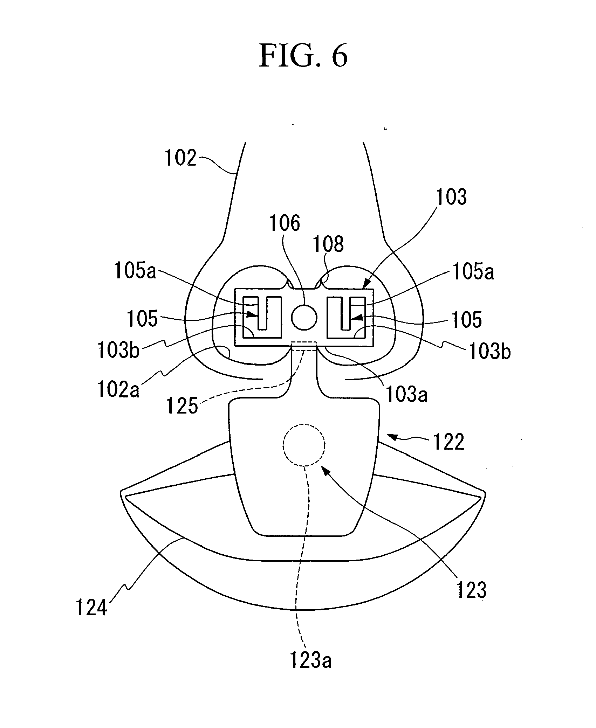

FIGS. 6 to 8 show the second embodiment of the present invention.
Parts in FIGS. 6 to 8 that are the same as the configuration elements mentioned in FIGS. 1 to 4, are assigned the same reference numerals and their descriptions are omitted.
The basic configuration of this embodiment is the same as the first embodiment mentioned above, and the differences are in the points mentioned below.
That is, as shown in FIG. 6, in this embodiment, the oral breath sensor 122 (sensor) extends below and is detachably installed to the nasal breath sensor 103 through a connector 125.
With the nasal breath sensor 103 attached to a specific member of the test subject, a pressure sensor for the mouth 123 (sensor) arranged at a position opposite to mouth 124, is fitted to the oral breath sensor 122. Also, the pressure sensor for the mouth 123, similar to the one mentioned above, is provided with piezoelectric element for the mouth 123a, the resistance value of which varies according to the displacement operation, and the direction of deformation and the deformation amount of this piezoelectric element for the mouth 123a are output as oral respiratory information.

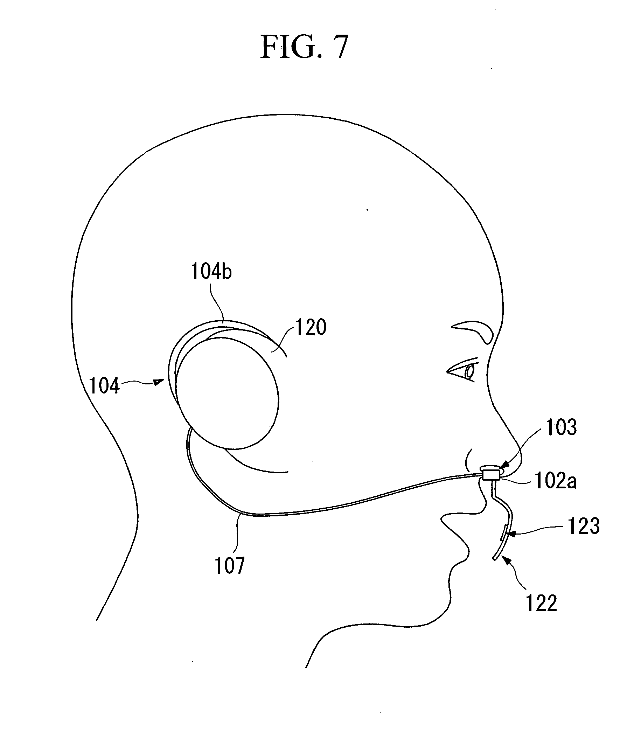

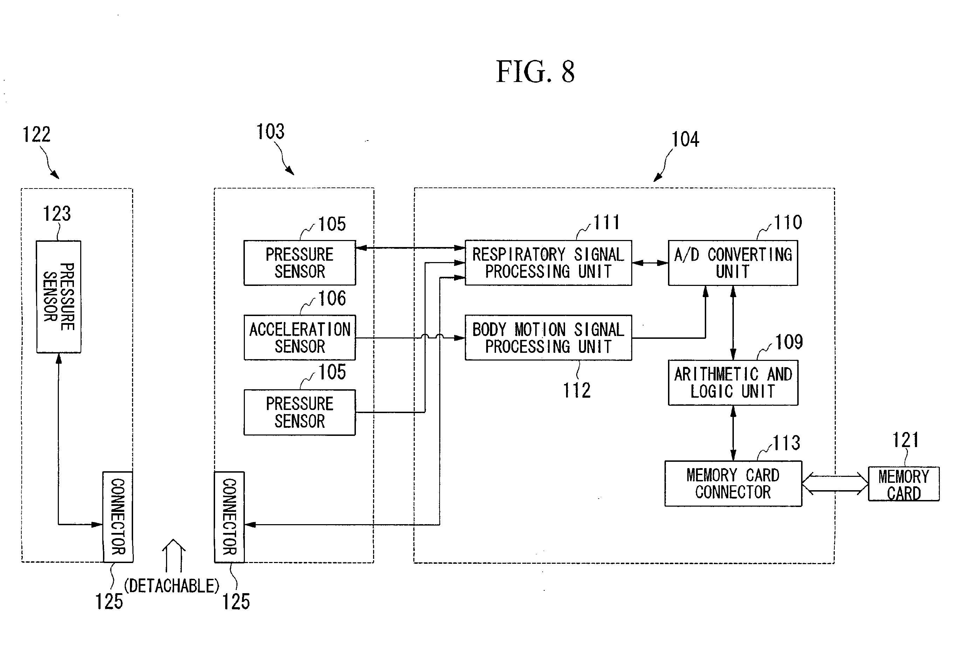

Based on the configuration mentioned above, the test subject attaches the nasal breath sensor 103 and the oral breath sensor 122, as shown in FIG. 7. Then, not only is the air breathed in from nostrils 102a detected, but also the respiratory air in the mouth 124 is detected by the oral breath sensor 122. The direction of pressure and strength of pressure of the airflow from the mouth 124 is likewise output as oral respiratory information by an action similar to that of the nasal breath sensor 103 from the oral breath sensor 122 as a signal. As shown in the block diagram of FIG 8, this output signal is input to the respiratory signal processing unit 111 through the connector 125. Furthermore, it is input to the arithmetic and logic unit 109 through the A/D converting unit 110, and subjected to specific arithmetic processing by the arithmetic and logic unit 109.
By the above, and according to the present embodiment, the oral respiratory information from the oral breath sensor 122 can be added in addition to the respiratory information from the nasal breath sensor 103. Therefore, even when a test subject breathes out air only from the mouth 124, the respiratory condition can be measured accurately.

In the first embodiment mentioned above, the nasal breath sensor 103 was attached to the nostrils 102a, but it is not limited to the nostrils only, and may be attached to the mouth of the test subject.
In the first embodiment and the second embodiment mentioned above, acceleration sensor 106 was installed at the center of the nasal breath sensor 103, but it is not limited to this location, and its installed location may be changed appropriately. Moreover, the nasal breath sensor 103 and the acceleration sensor 106 are installed as an integral unit, but each may be separately installed. In this case, the acceleration sensor 106, for instance, may be attached to the test subject's head and so on. However, it is understood that if both are installed as an integral unit, the respiratory condition can be measured with higher accuracy. Also, the acceleration sensor 106 was installed in the nasal breath sensor 103, but this acceleration sensor 106 may not necessarily be used. However, it is understood that if acceleration sensor 106 is installed, the respiratory condition can be measured with higher accuracy.
The data processing unit 104 is installed in the test subject's ear, but it is not limited to
this location, and the attached location or the installed location, may be appropriately changed.

### (Third embodiment)

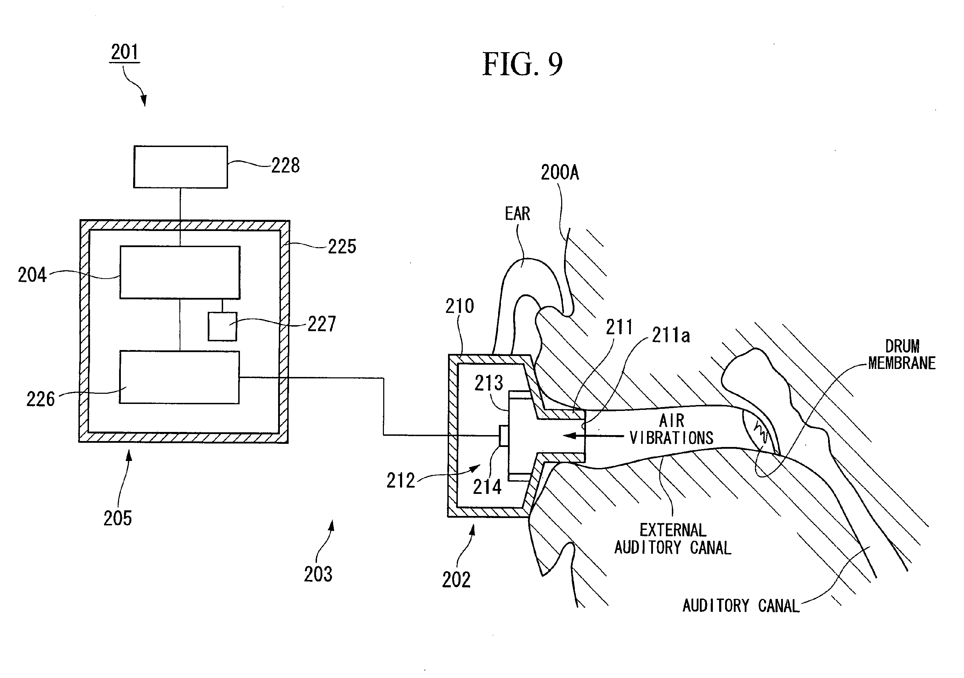

An embodiment of the device for measuring the respiratory condition related to the third embodiment of the present invention is described referring to FIGS. 9 to 13.
As shown in FIG. 9, the device for measuring the respiratory condition 1 of the present embodiment comprises an inserting unit (attaching unit) 211, a detecting unit 203, and the main body 205. The device for measuring the respiratory condition 201 is attached to the ear of test subject 200A through the inserting unit 211. The detecting unit 203 has a vibration sensor unit (vibration sensor) 202 for detecting the bio-signal corresponding to respiration. The main body 205 has a signal analyzing circuit (measurement means) 204 for measuring the respiratory condition based on the bio-signal detected by the detecting unit 203.
In the present embodiment, the air vibrations corresponding to respiration in test subject A's ear are explained as bio-signals mentioned above.

The vibration sensor unit 203 mentioned above, is shaped in the form of a box by a case 210. An inserting unit 211 that can be inserted in the external auditory canal, is installed in one end face of the case 210. That is, the vibration sensor unit 203, can be attached to the ear of the test subject 200A by inserting the inserting unit 211 in the external auditory canal. The inserting unit 211 is formed with an elastic material such as rubber so as to seal the external auditory canal when inserted in the external auditory canal, and also has an opening 211 a at the front end. Furthermore, the case 210 is formed of material that shuts off external sound and does not propagate sound within the case 210.
Within the case 210, a receiving means 212 is installed for receiving air vibrations in the ear. For instance, this receiving means 212 may be configured by attaching a crystal 214 to a thin film 213 provided within the case 210. The crystal 214 has the function of sending electric signal (waveform) corresponding to the vibrating condition of the thin film 213 to the main body 205 mentioned above, when the thin film 213 vibrates because of air vibrations in the ear.
As a result, the vibration sensor unit 203 can detect the air vibrations in the ear.

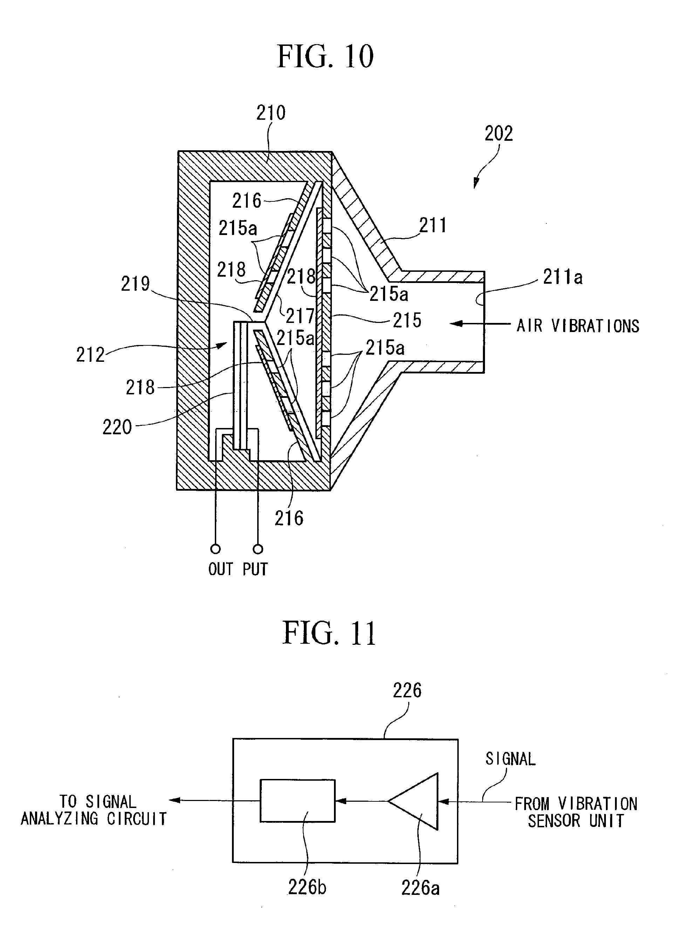

The receiving means 212 is not limited to the configuration mentioned above. For instance, the configuration shown in FIG. 10 is preferred. That is, an intermediate wall 215 formed with a plurality of very small openings 215a, and a slanting wall unit 216 that forms a V-shaped space with the intermediate wall, are provided on the side of the inserting unit 211 in the case 210. Similar to the intermediate wall 215, a plurality of openings 215a is formed in the slanting wall unit 216. A vibrating plate 217 formed in the shape of a V and made of a metal such as aluminum, is arranged in the space enclosed by the intermediate wall 215 and the slanting wall unit 216. An acoustic resistance 218 is arranged to cover the openings 215a on the inside of the intermediate wall 215 and the slanting wall unit 216.
A ceramic element 220 connected to the vibrating plate 217 through a rod 219, is fitted within the case 210. The ceramic element 220 may be a piezoelectric element, such as lead zirconate titanate (PZT), for instance, which generates charge corresponding to bending stress in structures called bimorph structures.
In this way, the vibrating plate 217 accurately picks up changes in vibration of low impedance air and vibrates, and electrical signals corresponding to these vibrations are sent by the ceramic element of the receiving means 212 to the main body 205.

The main body 205 mentioned above is formed in a box shape by the case 225, as shown in FIG. 9, and can be attached for instance, to the arm and so on of the test subject 200A by a belt and so on. It may also be configured such that it can be attached to the arm like a wrist watch. The main body 205 is connected electrically to the vibration sensor unit 202. The main body 205 includes a case 225 provided with a signal detection circuit 226, a signal analyzing circuit 204, and memory 227. The signal analyzing circuit 226 detects electrical signals sent by the above-mentioned receiving means 212. The signal analyzing circuit 204 measures the respiratory condition based on the electrical signals sent by the signal detection circuit 226. Memory 227 records the respiratory condition detected by the signal analyzing circuit 204. Furthermore, the outer surface of case 225 is provided with an indicator 228 for indicating various kinds of information recorded in memory 227.

The above-mentioned signal detection circuit 226 has an amplifying part 226a and a low pass filter 226b, as shown in FIG. 11. The amplifying part 226a amplifies the electrical signals sent by the receiving means 212.
The low pass filter 226b removes electrical signals due to heart rate, for instance; therefore, it removes unwanted signals that occur because of the heart rate of the test subject 200A that are included in the electrical signals amplified by the amplifying part 226a. That is, the signal detection circuit 226 and the above-mentioned vibration sensor unit 203 constitute the above-mentioned detecting unit 203.
The above-mentioned signal analyzing circuit 204 analyzes signals sent by the signal detection circuit 226, and determines whether the respiratory condition is normal or abnormal. For instance, it compares threshold values and so on that have been set beforehand, and if the signal level is greater than the threshold value, it determines the respiratory condition as abnormal; for instance, determines it as apneic condition. When the respiratory condition has been determined as abnormal, the signal analyzing circuit 204 sends this respiratory information to the memory 227.

The above-mentioned memory 227 has built-in timer functions, and it records the information on respiratory condition sent by the above-mentioned signal analyzing circuit 204 together with the time.
The above-mentioned indicator 228 is a monitor that can optionally indicate various
kinds of information recorded in the memory 227 for instance, by LED or by liquid crystal monitor using a switch not shown in the figures. In the present embodiment, the indicator 228 is installed on the outer surface of the case 225 and is a part thereof, but it is not limited to such a construction and it may be a separate unit.

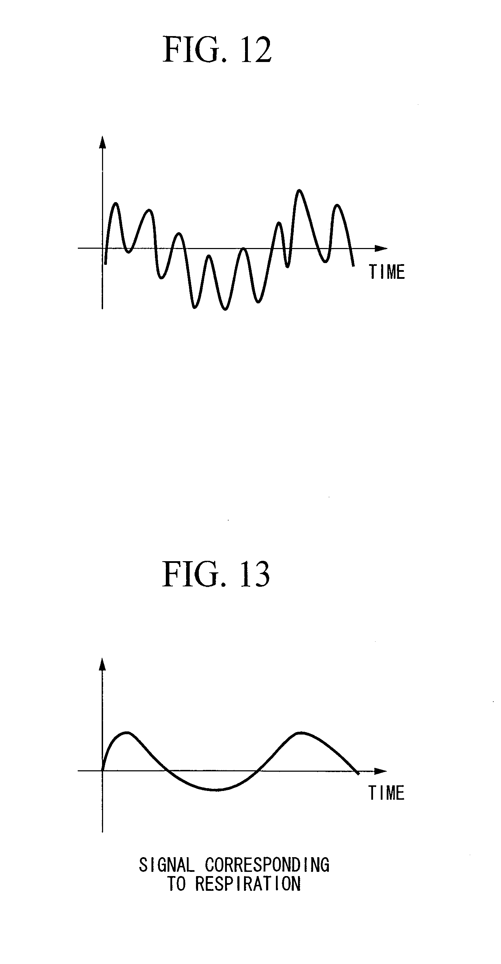

The detection of respiratory condition of the test subject 200A by the device for measuring the respiratory condition 201 configured as mentioned above, is described hereunder.
First, the vibration sensor unit 202 and the main body 205 are attached at the specified positions. After attachment, the test subject 200A turns on the power switch to the main body 205 not shown in the figures and goes to sleep. The vibration sensor unit 202 attached to the ear detects air vibrations in the ear in the frequency band from 0 KHz to 20 KHz, for instance, which correspond to respiration of the test subject 200A. That is, each time the test subject 200A breathes, changes in sound and pressure are propagated through the bones, the auditory tube, and so on, and the air in the auditory canal vibrates. These air vibrations enter the case 210 from the openings 211 a of the inserting unit 211 and are received by the receiving means 212. Also, when the receiving means 212 detects the air vibrations, it sends electrical signals corresponding to the vibrating condition, for instance, electrical signals of waveform as shown in FIG. 12, to the signal detection circuit 226. At this stage, the electrical signals sent to the signal detection circuit 225 include electrical signals generated by heart rate, for instance, in addition to the electrical signals generated by respiration.

The signal detection circuit 226 amplifies the electrical signals received using the amplifying part 226a, as shown in FIG. 11. Subsequently, electrical signals due to causes other than respiration as mentioned above, that is, electrical signals due to heart rate, are cut by the low pass filter 226b. As a result, the signal detection circuit 226 can acquire electrical signals corresponding to respiration, as shown in FIG. 13. Also, the signal detection circuit 226 sends the detected electrical signals to the signal analyzing circuit 204.
The signal analyzing circuit 204 compares the signal level of the sent electrical signals with the preset threshold values and the like. If the compared results show that the level lies below the threshold value, the respiratory condition is judged as normal. If the level is above the threshold level, the respiratory condition is judged as abnormal. For instance, it may be judged as apneic condition of test subject A and so on, and the memory 227 is notified the judgment.
The memory 227 records from time to time the sent information on respiratory condition together with the time using the timer function.

The device for measuring the respiratory condition 201 repeats the above-mentioned process until the power to the main body is cut off, and examines the respiratory condition of the test subject 200A during sleep.
After getting up, the test subject 200A can confirm easily various kinds of information recorded in the memory 227 by operating the switch of the indicator 228, such as, for instance, at what hour and minute changes in the respiratory condition (apneic condition) occurred, or the number of times the respiration was abnormal during the whole night.

According to the device for measuring the respiratory condition 201 mentioned above, changes in air vibrations in the ear corresponding to respiration can be detected by the vibration sensor unit 202, and based on the bio-signals corresponding to these air vibrations, the signal analyzing circuit 204 measures the respiratory condition of the test subject 200A. In this way, by merely attaching the vibration sensor unit 202 to the ear, the respiratory condition can be easily examined. Particularly, respiratory condition can be examined by attaching the vibration sensor unit 202 to the ear; therefore, the feeling of constraint is reduced and the probability of the unit coming off due to the rolling over and so on, is reduced in the test subject 200A. Moreover, the respiratory condition can be correctly detected even in a condition in which the effect of surrounding temperature or body motion is difficult to receive.
Furthermore, the signal detection circuit 226 removes unwanted signals that are generated by heart rate and the like, using the low pass filter 226b; therefore, accurate respiratory information can be acquired.

Note that the scope of the skill of the present invention is not limited to the embodiments mentioned above, and various changes may be effected to the present invention without departing from the spirit and scope of the present invention.

For instance, in the embodiment mentioned above, air vibrations in the ear (for instance, frequencies in the 0 KHz to 20 KHz band) were taken as bio-signals, but bio-signals are not limited to air vibrations only. For instance, air pressures in the ear corresponding to respiration may be taken as bio-signals. In this case, air pressure sensor that detects air pressure may be attached to the ear instead of the vibration sensor unit. Also, sounds in the ear corresponding to the respiration may be taken as bio-signals. In this case, sound sensor that detects sound may be attached to the ear instead of the vibration sensor unit. Particularly, the sound sensor is suitable for detection of vibrations with frequencies in the 20 Hz to 20 KHz band. Moreover, various kinds of information mentioned above, that is, combinations of air pressures and air vibrations corresponding to respiration may be used as bio-signals. In this case, a compound sensor that can detect such various kinds of information may be used.

Furthermore, acceleration sensor that detects body motion may be fitted to the vibration sensor unit. In this case, the acceleration sensor should preferably directly fitted on the inside of the case so that it does not affect the receiving means. The body motion information of the test subject 200A during sleep detected by the acceleration sensor may be sent to the signal detection circuit, and the signals after the low pass filter should preferably be corrected such that the effects of the body motion are eliminated. By adopting such a configuration, a more accurate examination of the respiratory condition of the test subject 200A can be performed.

The vibration sensor unit should preferably be attached to both ears instead of one of the ears of the test subject 200A. This will, for instance, enable average values of the bio-signals measured at both ears to be obtained even if the test subject 200A during sleep changes over to the posture of sleeping on the side after rolling over and so on, so that values are not susceptible to the effects of the posture. Accordingly, the accuracy of examination of respiratory condition improves.

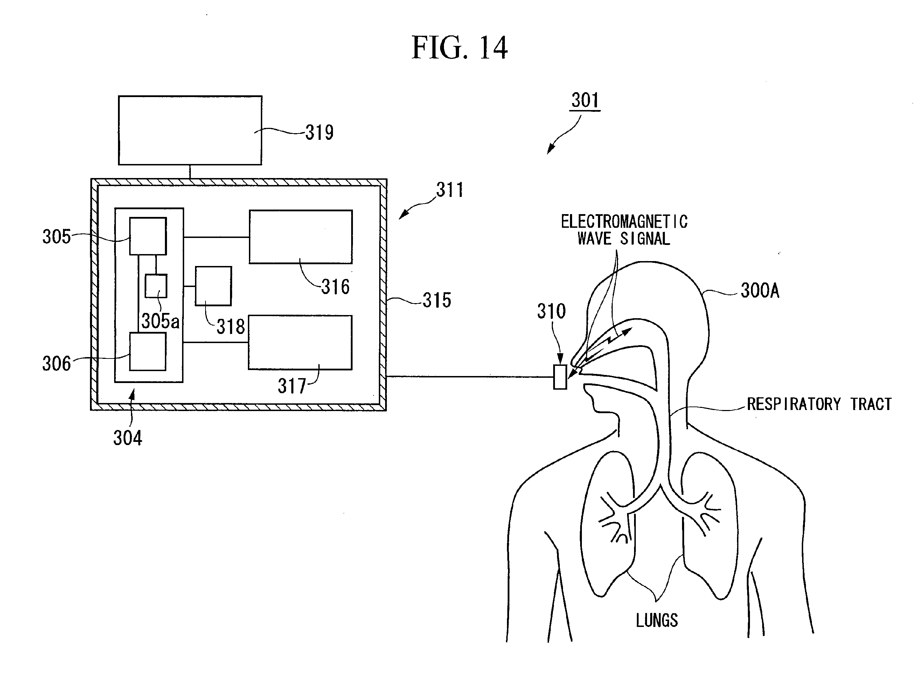

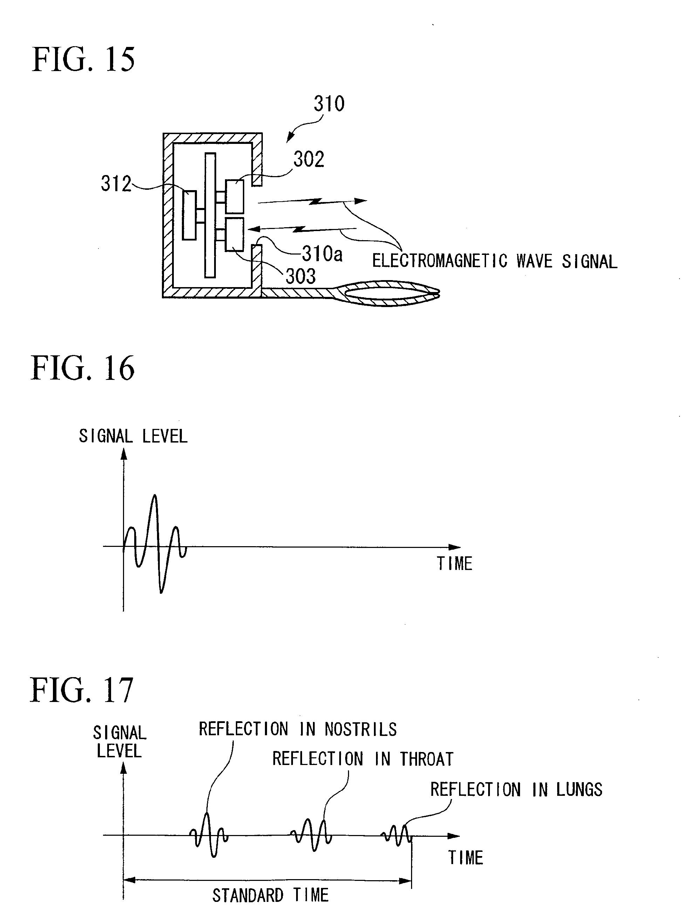

(Fourth embodiment)
Next, a device for measuring the respiratory condition related to the fourth embodiment of the present invention is described referring to FIGS. 14 to 23.
The device for measuring the respiratory condition of the present embodiment comprises a main body 311 and a sensor unit 310, as shown in FIG. 14. As shown in FIG. 15, the sensor unit 310 comprises an electromagnetic wave transmitter (transmitter) 302, an electromagnetic wave receiver (receiver) 303, and a radio wave antenna 312. The electromagnetic wave transmitter 302 transmits examination signals, that is, electromagnetic wave signals into the respiratory tract through the mouth and/or the nostrils of a test subject 300A. The electromagnetic wave receiver 303 receives the electromagnetic wave signals reflected from within the lungs or the respiratory tract. On the other hand, the main body 311 comprises a signal analyzing circuit 304 (pulse signal measurement unit), a signal generating circuit 316, and a signal detection circuit 317. The signal analyzing circuit 304 measures the respiratory condition of the test subject 300A based on the electromagnetic wave signals received by the electromagnetic wave receiver 303. In the present embodiment, the transmission and reception of electromagnetic wave signals in the respiratory tract from the nostrils are described.
The above-mentioned signal analyzing circuit 304 comprises an analyzing circuit (analyzing unit) 305 and a detecting circuit (detecting unit) 306. The analyzing circuit 305 analyzes the respiratory condition based on the measured results of electromagnetic wave signals. The detecting circuit 306 detects obstruction members in the respiratory tract, based on the analyzed results of the analyzing circuit 305.

As mentioned earlier, the device for measuring the respiratory condition 301 has a sensor unit 310 attachable to the part near the nostrils, and the main body 311 electrically connected to the sensor unit 310. The sensor unit 310 is shaped like a box with an opening 310a, as shown in FIG. 14 and FIG. 15. Holding units such as clips are provided such that the sensor unit can be attached to the test subject 300A with the opening 310a facing the nostrils through the holding unit. The holding unit may be omitted, and tape and the like may be used instead, to stick the sensor unit near the nostrils. Or, the outer shape may be in the form of a rugby ball, small dents and protrusions may be formed on its outer surface, and the sensor unit inserted and fixed within the nostrils. The sensor unit 310 also includes a radio wave antenna 12. This radio wave antenna 12 receives control signals from the main body 311, and operates the electromagnetic wave transmitter 302. The radio wave antenna 312 is arranged such that the electromagnetic wave receiver 303 sends electromagnetic wave signals received from the respiratory tract toward the main body 311. When transmitting electromagnetic wave signals, the electromagnetic wave transmitter 302 transmits the electromagnetic wave signals in pulse form and not as continuous transmissions.

The above-mentioned main body 311 is formed in the shape of a box by case 315, as shown in FIG. 14. It is attachable to the arm and the like of test subject 300A, for instance, by a belt and the like. It may also be configured such that it can be attached to the arm like a wrist watch.
As shown in FIG. 14, the main body 311 includes a case 315 containing a signal generating circuit 316 and a signal detection circuit 317. The signal detection circuit 316 generates control signals that are sent to the above-mentioned radio wave antenna 312.
The signal detection circuit 317 detects electromagnetic wave signals transmitted by the radio wave antenna 312.

Furthermore, the main body 311 includes the above-mentioned signal analyzing circuit 304 provided in the case 315. This signal analyzing circuit 304 includes an analyzing circuit 305, a detection circuit 306, and a memory 318. The memory 318 records the detected results of members such as obstruction members and the respiratory condition obtained in each of these circuits 305 and 306. The outer surface of the case 315 includes an indicator 319 that indicates various kinds of information recorded in the memory 318. The above-mentioned analyzing circuit 305 includes the function of measuring the reflection time of an electromagnetic wave signal transmitted from the electromagnetic wave transmitter 302 and reflected in the respiratory tract until it is received by the electromagnetic wave receiver 303. The respiratory condition and the obstruction condition in the respiratory tract are analyzed according to the difference in the reflection times. Moreover, the analyzing circuit 305 includes a recording unit 305a, which records the reflection time during the interval from the start of the operation to a specific time, that is, the reflection time immediately measured after the test subject 300A has gone to sleep, as the standard time. Then, analysis is performed by comparing the standard time and the reflection time measured later. The recording unit 305a may be set such that standard time can be input beforehand. If the measured reflection time from the analyzed results and the standard time differ, the difference is treated as a change in the respiratory condition, transmitted to the detection circuit 306, and recorded in the memory 318.

The above-mentioned detection circuit 306 has the function of detecting the distance from the reflection time sent by the analyzing circuit 305 to the position of obstruction in the respiratory tract. The detected distance is recorded in the memory 318.
The above-mentioned indicator 319 is a monitor that can optionally indicate various
kinds of information recorded in the memory 318 for instance, by LED or by liquid crystal monitor using a switch not shown in the figures. The indicator 319 has a built-in timer function, and it can record the times of reception of various kinds of information sent by the analyzing circuit 305 and the detection circuit 306. In the present embodiment, the indicator 319 is installed on the outer surface of the case 315 and is a part thereof, but it is not limited to such a construction and it may be a separate unit.

The detection of respiratory condition and position of obstruction in the respiratory tract of the test subject 300A by the device for measuring the respiratory condition 301 configured as mentioned above, is described hereunder.
First, the sensor unit 310 and the main body 311 are attached at the specified positions. After attachment, the test subject 300A turns on the power switch to the main body 311 not shown in the figures and goes to sleep. Now, when power is supplied to the main body 311, the electromagnetic wave transmitter 302 transmits the electromagnetic wave signals to the respiratory tract. That is, the signal generating circuit 316 activates, and generates the specified control signal. Based on this control signal, pulse-type electromagnetic wave signals are transmitted from the electromagnetic wave transmitter 302 into the nostrils, as shown in FIG. 16. At this stage, the respiratory tract of the test subject 300A is not obstructed because the condition is just after sleep. Accordingly, the electromagnetic wave signals transmitted to the respiratory tract reach the lungs after being reflected by the lumen wall in the respiratory tract of test subject A, as shown in FIG. 14. They are
received by the electromagnetic wave receiver 303 after they return, and are reflected by the lungs toward the nostrils again. The received electromagnetic wave signals are sent to the main body 311 through the radio wave antenna 312. Then they are sent from the signal detection circuit 317 to the signal analyzing circuit 304.

In this case, the electromagnetic wave receiver 303 receives the electromagnetic wave signals from the respiratory tract, for instance, and sends them to the analyzing circuit 305, as shown in FIG. 17. That is, the electromagnetic wave receiver 303 first receives the electromagnetic wave signals reflected by the lumen wall in the nostrils, and then receives the electromagnetic wave signals reflected by the lumen wall in the throat. In this way, with the passage of time, electromagnetic wave signals reflected by the lumen walls of members close to the lungs are received. Finally, the electromagnetic wave signals reflected by the lungs are received. The analyzing circuit 305 judges the electromagnetic wave signal just before the signal level becomes zero as the electromagnetic wave signal reflected from the lungs. Also, the analyzing circuit 305 records the reflection time of the electromagnetic wave signal reflected from the lungs as the standard signal in the recording unit 305a. The recording unit 305a is set such that recording is performed only for the duration of a specified time after the power has been switched on. The average value of the standard time after recording several times during the specified duration may be determined also. In this way, standard time can be set more accurately.

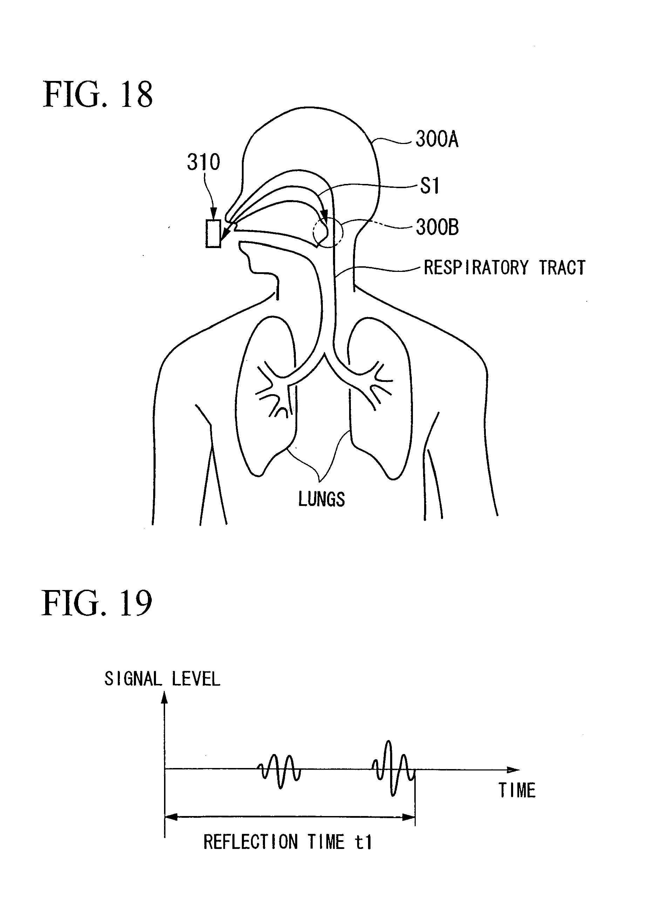

Next, after the specified time mentioned above (for instance, 30 minutes from the time of switching on the power) has elapsed, the analyzing circuit 305 analyzes the respiratory condition by comparing the reflection time of the electromagnetic wave signal sent by the electromagnetic wave receiver 303 with the standard time. The result of the analysis if there is no difference in the reflection time, for instance, is that there is no change in the condition of the respiratory tract; that is, there is no change in the respiratory condition. If the muscles of the respiratory tract of the test subject 3001 have become slack, for instance, and if a constriction has occurred between the nostrils and the mouth, the electromagnetic wave receiver 303 receives electromagnetic wave signals as shown in FIG. 19. Stated differently, the electromagnetic wave receiver 303 receives the electromagnetic wave signals reflected at the constricted member 300B and not the lungs, that is, receives the electromagnetic wave signals of reflection time t1. The analyzing circuit 305 analyzes the change in the respiratory condition from the difference between this reflection time t1 and the standard time, and sends this result to the memory 318 and the detection circuit 306.

The memory 318 records the change in the respiratory condition from the analyzing circuit after associating the time according to the built-in timer function with this change. The detection circuit 306 also detects the distance s1 to the obstruction member 300B from the reflection time t1 sent by the analyzing circuit 305. Stated differently, the detecting circuit 306 detects the distance s1 to the obstruction member 300B from the reflection time t1 based on the following formula, for instance: Distance S (mm) = Time (s) x speed of sound (m/s) /2. The detection circuit 6 sends the distance s 1 after detection
to the memory 318. The memory 318 records the distance s1 sent by the detection circuit 6 after associating it with the time of the built-in timer function.

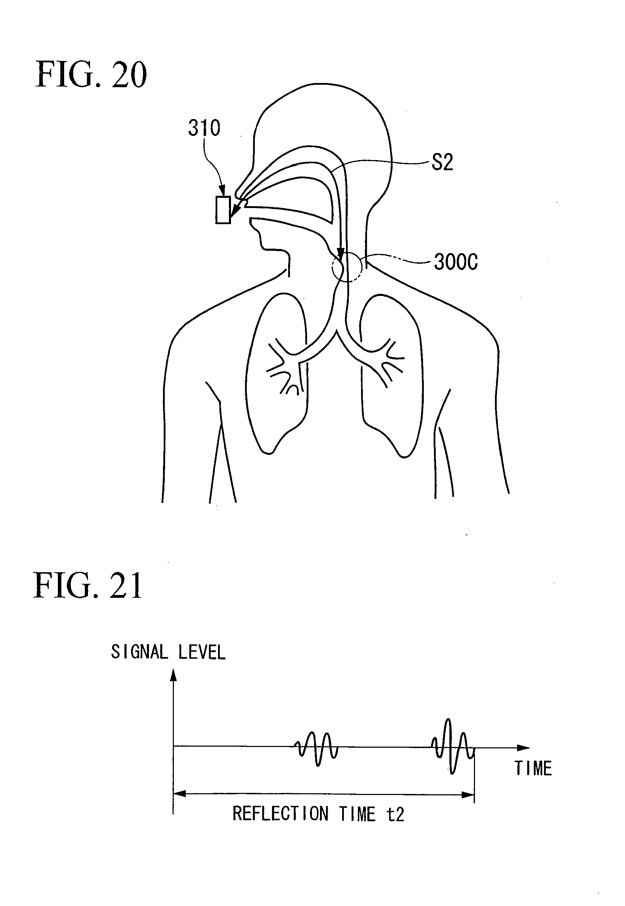

Also, as shown in FIG. 20, if a constriction occurs between the lungs and the mouth of the respiratory tract, for instance, the electromagnetic wave receiver 303 receives the electromagnetic wave signals as shown in FIG. 21, that is, receives the electromagnetic wave signals of reflection time t2. In this case, similar to the case mentioned above, the change in the respiratory condition is analyzed in the analyzing circuit, and the distance s2 to the obstruction member 300C is detected by the detection circuit 306. The changes in this respiratory condition and the distance s2 are recorded after associating them with time in the memory 318.

In this way, when the test subject 300A is asleep, the signal analyzing circuit 304 receives the electromagnetic wave signals transmitted by the electromagnetic wave transmitter 302 and reflected in the respiratory tract. When a difference between the reflection time and the standard time occurs, it treats this difference as a change in the respiratory condition and repeatedly records the change in the memory 318. At the same time, it also records the distance to the obstruction member.
As a result, after the test subject 300 lies down, various kinds of information recorded in the memory 318, such as, at what time the change in the respiratory condition (apneic condition) occurred, distance from the nostrils to the obstruction member, and the number of times the breathing was abnormal, and so on, can be easily confirmed by turning on the switch of the indicator 319. Particularly, the distance from the nostrils to the obstruction member can be confirmed; therefore, appropriate medical treatment can be received immediately without receiving treatment such as MRI from a medical institution subsequently.

According to the above-mentioned device for measuring the respiratory condition 301, by transmitting electromagnetic wave signals to the respiratory tract of the test subject 300A, and by receiving the electromagnetic wave signals reflected from the respiratory tract, the position of obstruction in the respiratory tract can be easily detected without expending time or effort, and the respiratory condition can also be examined. Accordingly, medical treatment subsequently, such as the treatment for the apneic condition during sleep by medical institutions and the like, can be performed smoothly based on the data of the examined obstruction position.
Since the electromagnetic wave transmitter 302 and the electromagnetic wave receiver 303 are disposed near the nostrils, the electromagnetic wave signals can be transmitted into and received from the respiratory tract correctly. Accordingly, the accuracy of the examination can be improved.

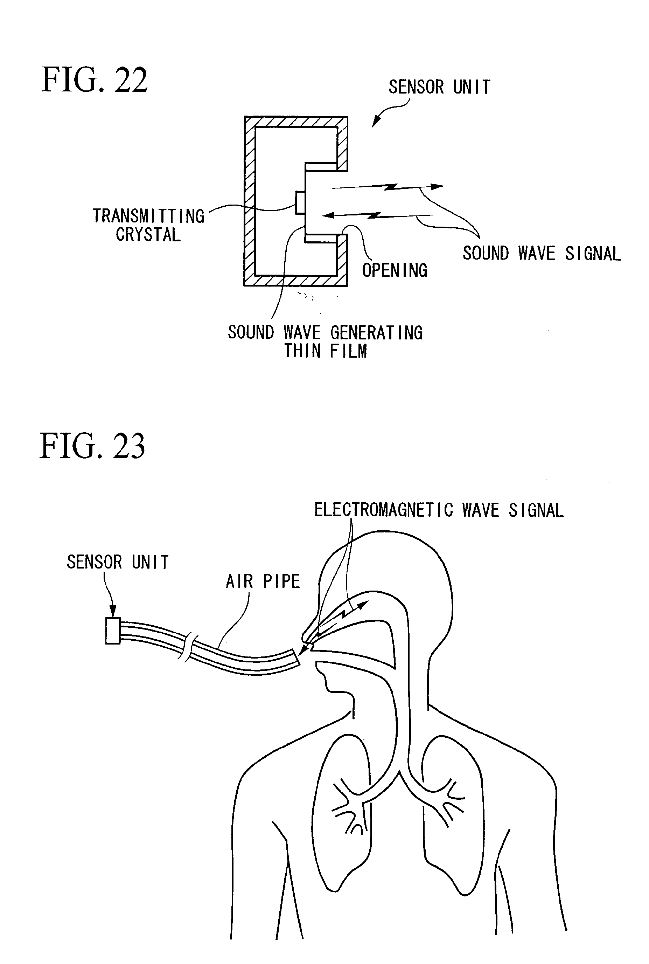

Note that the scope of the skill of the present invention is not limited to the embodiments mentioned above, and various changes may be effected to the present invention without departing from the spirit and scope of the present invention.
For instance, in the above-mentioned embodiment, the electromagnetic wave signals
were transmitted from the nostrils of the test subject to the respiratory tract, but they may be transmitted from the mouth to the respiratory tract. Although electromagnetic waves were used as the examination signals, the scope of this invention is not limited to electromagnetic waves only. For instance, sound wave signals using sound waves may also be used. In this case, the electromagnetic wave transmitter may be configured as the sound wave transmitter transmitting sound wave signals, and the electromagnetic wave receiver may be configured as the sound wave receiver that receives sound wave signals. As shown in FIG 22, a sound wave generating thin film may be provided near the opening in the sensor unit, and transmission means such as a transmitting crystal for vibrating the sound wave generating thin film by applying voltage on this sound wave generating thin film may be provided. By this configuration, the sound wave generating thin film can be vibrated and audio wave signals can be transmitted to the respiratory tract. Conversely, the audio wave signals from the respiratory tract can be received.

Also, the electromagnetic wave transmitter and the electromagnetic wave receiver may be separately configured, that is, the signal transmitter and the signal receiver were separately configured in the case of the main body, but they may be configured on the same device. By such an arrangement, the number of parts can be reduced and the cost can be reduced. The main body can also be made more compact.
Furthermore, the sensor unit was arranged near the nostrils, and electromagnetic wave signals were directly transmitted from the radio wave antenna to the respiratory tract, but the invention is not limited to this; any configuration that transmits electromagnetic wave signals to the respiratory tract may be used. As shown in FIG. 23, an air pipe (examination signal propagation guide) that can propagate the electromagnetic wave signals may be disposed between the sensor unit and the respiratory tract, for instance. This air pipe may be formed to have flexibility, and propagate electromagnetic wave signals internally. By using this air pipe, the sensor unit need not be attached to the test subject by adhesive or the like; therefore, the feeling of constraint of the test subject can be reduced.

### (Fifth embodiment)

The fifth embodiment for carrying out the invention is described here in detail referring to the drawings.

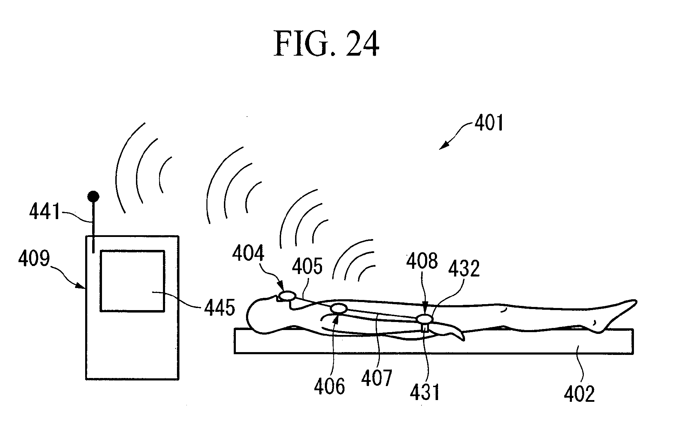

FIG. 24 shows the schematic configuration drawing of the device for measuring the respiratory condition during sleep in the fifth embodiment.
As shown in FIG. 24, the device for measuring the respiratory condition during sleep 401 comprises a detecting device 404 (detecting unit), a communication switching means 406, a transmitting device 408, and a receiving device 409. The detecting device 404 is attached to the head of a test subject lying on the side, and it detects the respiration of the test subject. The communication switching means 406 is connected by cable 405. The transmitting device 408 is connected by cable 407. The receiving device 409 can perform radio communication with the transmitting device 408.

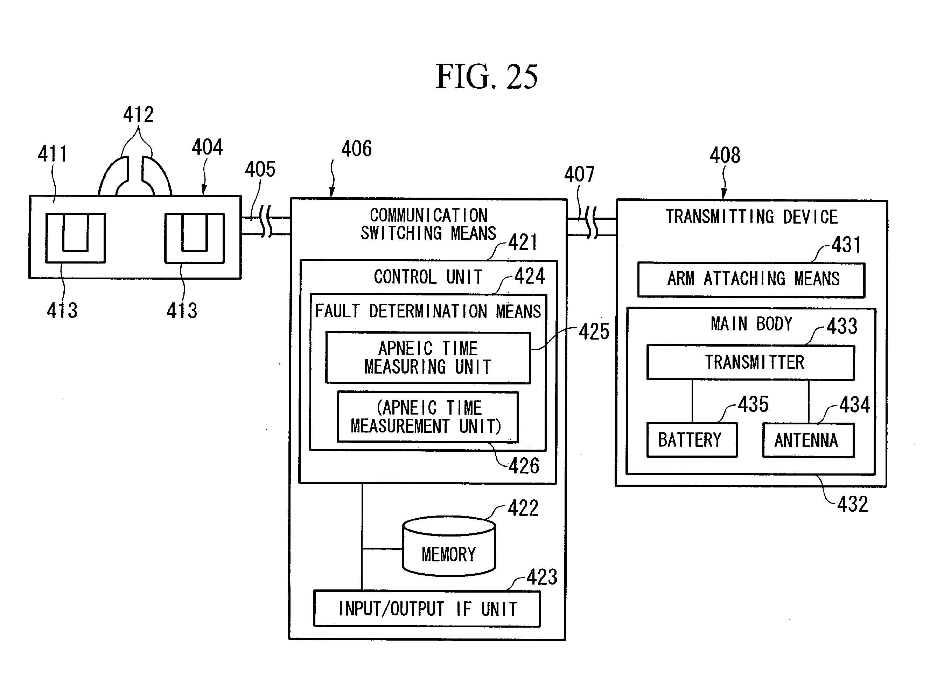

As shown in FIG. 25, the detecting device 404 has a slender-shaped main body 411, with a stopper 412 fitted near the center in the longitudinal direction of the main body 411 for fixing the main body 411 to the bridge on the nose of the test subject. Nasal breath sensors 413 are fitted one each on the left and right sides of the stopper 412 to the main body. The nasal breath sensors 413 are fitted to suit the formed position of each nostril. For instance, sensors fitted with piezoelectric element in the beam that deforms by nasal breath, or sensors to detect carbon dioxide and so on, may be used. From each of the nasal breath sensors 413, signals (detection signals) the strength of which varies with the respiration amount are output as detection signals indicating the respiratory condition of the test subject. These signals are output to the communication switching means 406 connected by the cable 405.

The communication switching means 406 comprises a control unit 421 made of a central processing unit (CPU), memory for recording data 422, and an input/output interface (IF) unit 423 for input/output of signals to cables 405 and 407. Furthermore, the control unit 421 includes fault determination means 424, which determines whether respiration is normal or abnormal according to determination algorithms mentioned later, based on the data output from detecting device 404. This fault determination means 424 includes an apneic time measuring unit 425. The apneic time measuring unit 425 measures the time for which the apneic condition continues.

As shown in FIG. 24 and FIG. 25, the transmitting device 408 includes the main body
432 and the arm attaching means 431.
The main body 432 is secured to the test subject's wrist through the arm attaching means 431. However, the main body 432 is made detachable by the arm attaching means 431. This main body 432 is connected to the communication switching means 406 and the detecting device 404 through the cable 407. The main body 432 includes a transmitter 433, an antenna 434, and a battery 435. The transmitter 433 and the antenna 434 constitute the radio communication means. This radio communication means superimposes the detected signals output from the communication switching means 406 on the electromagnetic waves (transmission waves) and transmits them. A belt with a stopper or surface fastener fitted to it, or a ring-shaped belt that can be freely extended can be used as the arm attaching means 431.

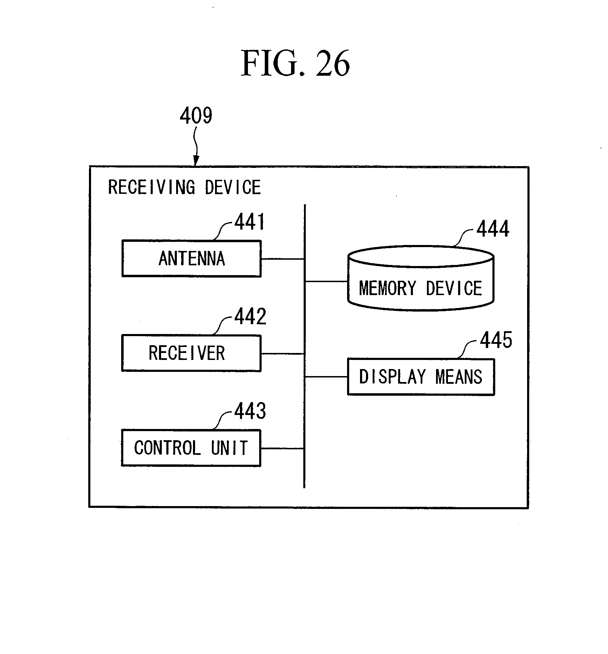

All devices from the above-mentioned detecting device 404 to the transmitting device 408 are fitted on the side of the test subject. The signals transmitted from the transmitting device 408 are transmitted using radio communications skills in the receiving device 409 installed at a position distant from the test subject.
As shown in FIG 26, the receiving device 409 includes an antenna 441 and a receiver 442. This antenna 441 and the receiver 442 constitute the radio communication means. This radio communication means receives electromagnetic waves transmitted by the transmitting device 8, and demodulates the detected signals. Furthermore, the control unit 443 comprising CPU and so on, and the recording device 444 that records data such as detected signals, include display means 445, such as liquid crystal display. The display means 445 displays graphs of detected signals, graphs indicating respiratory condition, and results of determination of faults in respiration, and so on. The determination of respiratory condition is made in the control unit 443 based on the detected signals.

Next, the processes in the device for measuring the respiratory condition during sleep
401 are described here referring mainly to FIGS. 24 to 26.
First, the detecting device 404 is attached to the test subject's nose, the communication switching means 406 such as belt (not shown in the figures) is fitted to the arm, and the transmitting device 408 is fitted to the wrist. Also, the receiving device 409 is installed at a position distant from the bed 402 but within the reach of electromagnetic waves from the transmitting device 408. In this condition, when the test subject breathes through the nose, detection signals corresponding to respiration are output from the nasal breath sensor 413 to the communication switching means 406.
The communication switching means 406 saves the detected signals in memory 422 only for a fixed period of time, and it determines the occurrence of a respiratory fault by the fault determination means 424.

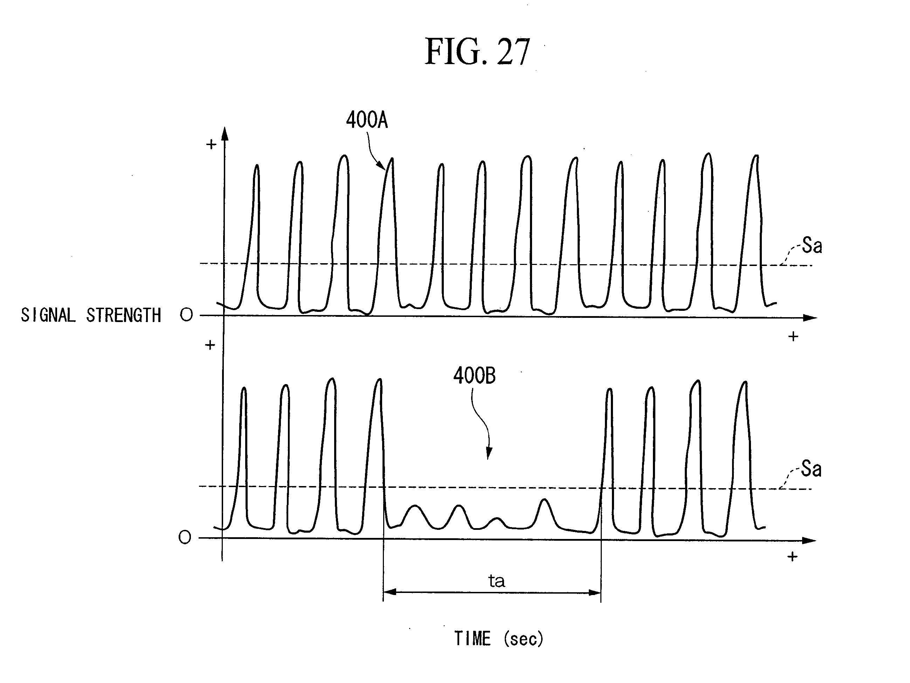

An example of the determination algorithm of the fault determination means 424 is described here referring to FIG. 27. The horizontal axis of FIG. 27 shows the time elapsed, while the vertical axis shows the strength of detected signals output by the nasal breath sensor 413 corresponding to the test subject's respiration. At each peak, the area from the rise to the peak position (apex) corresponds approximately to breathing in air, while the area from the peak position to the point where it drops to noise level corresponds approximately to the breathing out of air.

As shown by the reference numeral 400A in FIG. 27, when respiration is normal, a convex-shaped waveform of approximately the same shape as during normal respiration occurs cyclically. Thus, the peak of this convex-shaped waveform also occurs cyclically. In contrast, when abnormal respiration occurs, a waveform that does not exceed the specified threshold value (apnea determination standard Sa) occurs after a fixed time, as shown by the reference numeral 400B. For this reason, the peak occurrence cycle becomes disturbed in this interval. The time when abnormal respiration continues is taken as the time from the drop of the peak after crossing the apnea determination standard Sa to the rise of the peak after crossing the apnea determination standard Sa, and this apneic time is taken as ta. This apneic time ta is measured by the apneic time measuring unit 425 of the fault determination means 424.
When this apneic time ta exceeds the determination time set in the memory beforehand (for instance, 10 seconds), respiration fault is judged.
It should preferably also detect the occurrence of changes in the respiration of the test subject, even if such changes do not become faults. Therefore, the apnea determination standard Sa is set at a value higher than the signal strength corresponding to the actual apnea.

If the fault determination means 424 determines a respiration fault, the communication switching means 406 outputs a signal (send command signal) to the transmitting device 408 instructing it to send the detected signal. From the detected signals stored temporarily in the memory 422, the signal corresponding to respiration fault is output to the transmitting device 408. When the transmitting device 408 receives the send command signal, it superimposes the detected signal on the wave to be transmitted, and sends it to the receiving device 409 with the specified output. The transmitting device 408 does not send the electromagnetic wave if the send command signal and the detected signal have not been input. The communication switching means 406 outputs only the detected signal and does not output the send command signal. The transmitting device 8 may automatically transmit the detected signal if it acquires it.

The receiving device 409 that receives the electromagnetic waves mentioned above, demodulates the detected signals, and analyzes the data in the control unit 443. More specifically, the graphs of the detected signals are plotted, and output to the display means 445. The receiving device 409 can determine whether the respiration is normal or abnormal from the profile of the detected signal, and can also display the determined results. The results of data processing and the detected signals are recorded in the receiving device 409, and the detected signals can be confirmed later.

According to the fifth embodiment, the detecting device 404 to detect respiration during sleep was attached on the side of the test subject, a device for processing data (receiving device 409) of the detected signals was installed at a location distant from the test subject, and the detected signals were acquired using radio communication, thus enabling the feeling of constraint in the test subject to be reduced significantly.
Moreover, the communication switching means 406 was provided between the detecting device 404 and the transmitting device 408, respiration fault (including risk of respiration fault; same hereafter) was judged from the changes in the detected signals, and only when respiration fault was determined, the detected signals were transmitted by the transmitting device 408. Therefore, the time for transmitting the detected signals by the transmitting device 408 can be reduced, and the life of the battery 435 of the transmitting device 408 (see FIG. 25) can be prolonged.

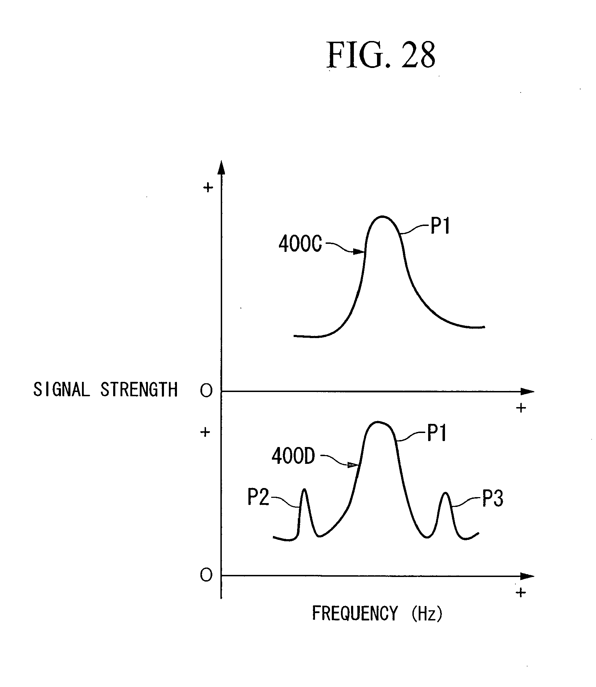

The embodiments of the present invention can be applied in various ways.
For instance, the respiratory cycle measuring unit 426 may be used in the fault determination means 424 shown in FIG. 25, instead of the apneic time measuring unit 425. The frequency components of the detected signals may be examined, and respiratory fault can be determined from the respiratory cycle (frequency) by this respiratory cycle measuring unit 426. The processes for determining such cases are described here referring to FIG. 27 and FIG. 28.
First, peaks occur at approximately fixed cycles as mentioned above, during normal respiration as shown by the reference numeral 400A in FIG. 27. Consequently, if these detected signals are subjected to Fourier transformation using the respiratory cycle measuring unit 426, a profile having one peak at the center of specific frequencies can be obtained, as shown by the reference numeral 400C in FIG. 28. In contrast, when abnormal respiration occurs as shown by the reference numeral 400B of FIG. 27, and if the detected signals are subjected to Fourier transformation, then a profile as shown by reference numeral 400D of FIG. 28 is obtained. This profile has small frequency peaks P2 and P3 equivalent to the disturbed breaths in the abnormal condition superimposed on specific frequencies equivalent to breaths in the normal condition with peak P1. That is, if the detected signals are subjected to Fourier transformation and the number and position of frequency peaks are studied, then the existence of occurrence of abnormal breaths can be determined. If this determination algorithm is used, the evaluation is made by the frequency of the respiration; therefore, the amplitude of the detected signals is not susceptible to fluctuations. Here, the fault determination means 424 may be provided with the apneic time measuring unit 425 and the respiratory cycle measuring unit 426. In this way, the accuracy of fault determination can be improved further.

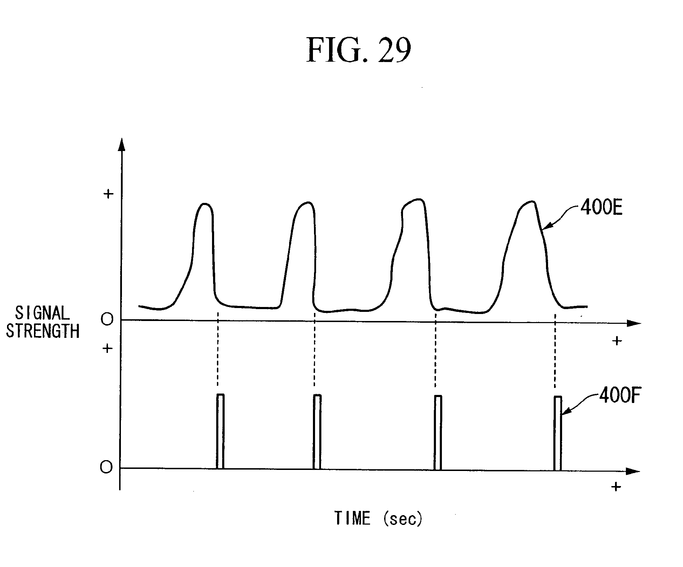

Also, the communication switching means 406 may be provided with the pulse-type signal generating means (not shown in the figures) for generating pulse-type signals from the detected signals. As shown in FIG. 29, this pulse type signal generating means generates pulse-type signals as shown by reference numeral 400F corresponding to detected signals as shown by reference numeral 400E. More specifically, when peaks of the detected signals occur with the respiration of the test subject, a pulse signal with height proportional to the amplitude of the detected signal is generated each time the peak falls. The width of this pulse signal is controlled so that it becomes smaller than the half-value width of the peak of the detected signals.
The receiving device 409 receives such pulse-type signals as signals indicating the respiratory condition of the test subject. Also, the receiving device 409 can determine whether the respiration of the test subject is normal or abnormal from the occurrence cycle and pulse height. Such a device for measuring the respiratory condition during sleep 401 can reduce the amount of radio communication information since the transmitting device 408 transmits pulse-type signals only once for one respiration cycle to the receiving device 409. Consequently, the life of the battery 435 of the transmitting device 408 can be prolonged.
Since the amount of radio communication information can be reduced with pulse-type signals, pulse-type signals corresponding to the detected signals may be transmitted not only when the fault determination means 424 of the communication switching means 406 has judged apnea, but also when the respiration is normal. Also, the pulse type signal generating means may be provided with transmitting device 408 instead of the communication switching means 406.

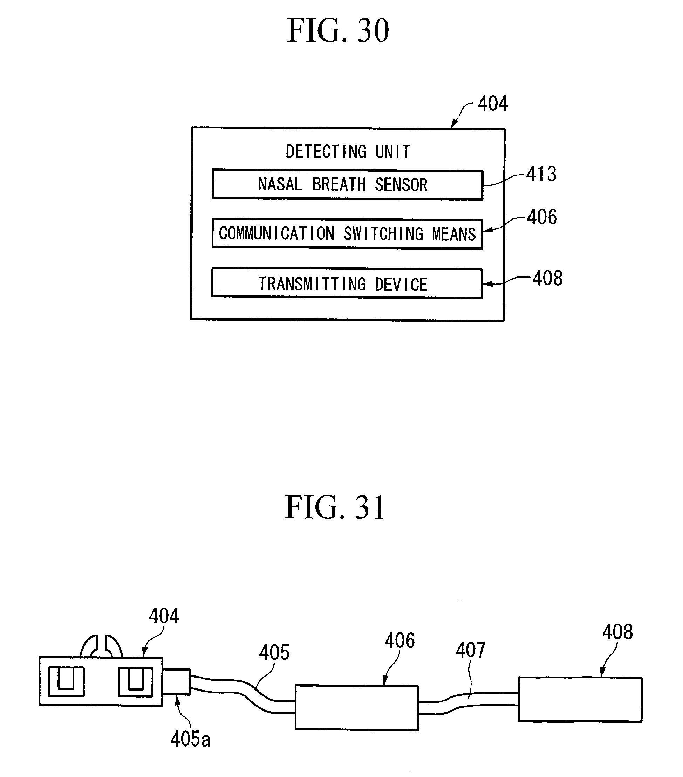

Moreover, the device for measuring the respiratory condition during sleep 401 may include the detecting device 404, the communication switching means 406, and the transmitting device 408 as one unit. For instance, as shown in FIG. 30, the detecting device 414 includes two nasal breath sensors 413 corresponding to the nostrils, a communication switching means 406, and a transmitting device 408. Here, the transmitting device 408 comprises the transmitter 433, the antenna 434, and the battery 435 shown in FIG. 25. Also, this detecting device 414 has at least one of the following means for attaching it to the test subject: the stopper 412 shown in FIG. 24 or a band for attaching it to the head.
According to this device for measuring the respiratory condition during sleep 401, since the detecting device 404, the communication switching means 406, and the transmitting device 408 were integrated as one unit, and attached below the nose of the test subject, the cables 405 and 407 became unnecessary, and the feeling of constraint in the test subject was further reduced. The life of the battery 435 can be prolonged as mentioned above, by associative operation of the communication switching means 406 and the transmitting device 408.
Even in a device for measuring the respiratory condition during sleep not having a communication switching means 406, the detecting device 404 and the transmitting device 408 may be integrated in one unit. In this case also, the same effect as mentioned above can be obtained.

Also, as shown in FIG. 31, a means for attachment/removal 405a may be provided in the cable 405 connecting the detecting device 404 and the communication switching means 406 for a configuration that permits the cable 405 to be freely attached/removed from the detecting device 404. The means for attachment/removal 405a is provided at the front end of the cable 405 on the side of the detecting device 404. This means for attachment/removal 405a comprises of members such as clips for engaging cable 405 and detecting device 404, and members such as screws for screwing the cable 405 in the detecting device 404.
According to this device for measuring the respiratory condition during sleep 401, by providing means for attachment/removal 405a, the detecting device 404 can be dissociated from the communication switching means 406 and the transmitting device 408. Consequently, in case of damage to the detecting device 404, it can be replaced easily. Moreover, by making the detecting device 404 disposable, it can be disinfected and re-used, and thus detecting device 404 can be maintained in a clean condition at all times. The communication switching means 406 and the transmitting device 408 can be kept permanently attached to the test subject, and the detecting device 404 can be connected when necessary; therefore, the load on the test subject can be reduced. On the other hand, if the detecting device 404 is kept permanently attached, and if the communication switching means 406 and the transmitting device 408 are connected when necessary, the communication switching means 406 and the transmitting device 408 can be shared between a plurality of test subjects. As mentioned earlier, the life of the battery can be prolonged by associative operation of the communication switching means 406 and the transmitting device 408.

The device for measuring the respiratory condition during sleep may be configured without the communication switching means 406, that is, the detecting device 404 may be directly connected to the transmitting device 408 by cable 407. Even in such a configuration, arrangement and configuration similar to the above-mentioned means for attachment/removal 405a may be installed in the cable 407 so that the detecting device 404 and the transmitting device 408 can be freely attached and removed. In this case also, the detecting device 404 can be kept in a clean condition at all times, and the transmitting device 408 can be shared.
The detecting device 404 and the communication switching means 406 may be formed as one unit, and these may be connected to the transmitting device 408 by the cable 407. In this case also, means for attachment/removal similar to the arrangement and configuration as the means for attachment/removal 405a may be provided in the cable 407. In this way, the actions and effects mentioned above can be obtained.

### (Sixth embodiment)

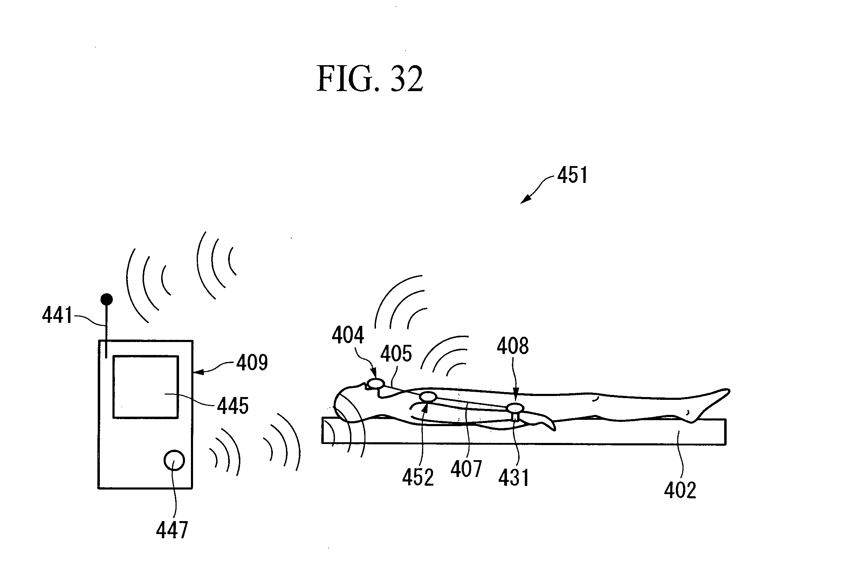

Next, the sixth embodiment for carrying out the invention is described here in detail referring to the drawings. The same reference numerals are affixed to elements with the same configuration as in the fifth embodiment, and explanations that duplicate those in the above-mentioned fifth embodiment are omitted here.
As shown in FIG. 32, the device for measuring the respiratory condition during sleep 451 is characterized in that it adjusts the output of electromagnetic waves transmitted by the transmitting device 408. For such adjustments, the transmission output adjusting means 452 and the receiving device 409 are used.

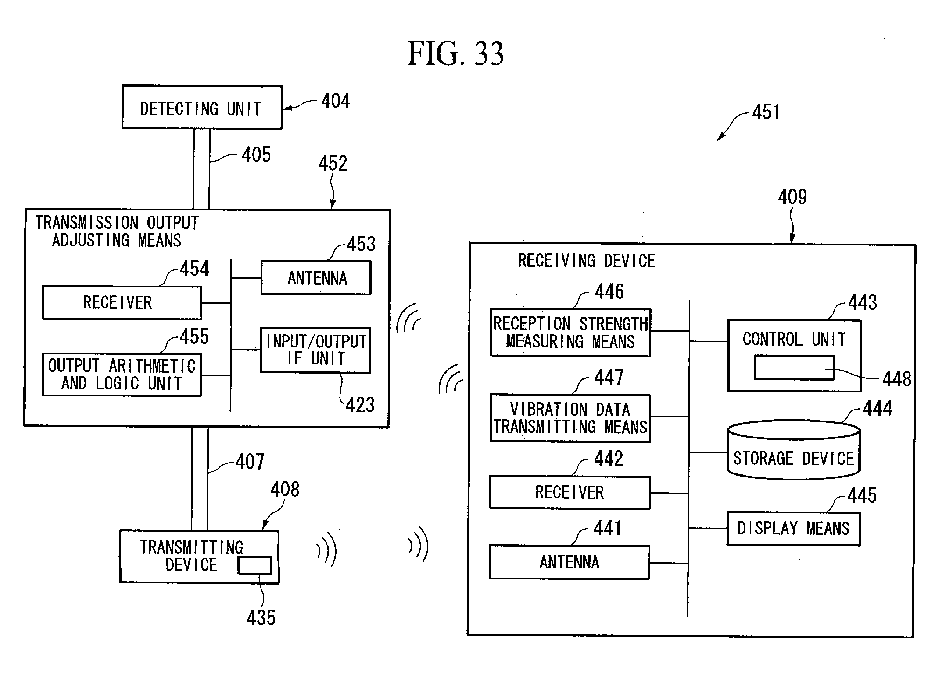

As shown in detail in FIG. 33, the transmission output adjusting means 452 is connected to the detecting device 404 by cable 405, and is connected to the transmitting device 408 by cable 407. More specifically, it comprises the radio communication means, an output arithmetic and logic unit 455, and an input/output interface unit 423. The radio communication means comprises an antenna 453 for receiving data (amplitude data mentioned later) transmitted by the receiving device 409, and a receiver 454. The output arithmetic and logic unit 455 arithmetically processes the electromagnetic wave output transmitted by the transmitting device 408, based on the amplitude data.

The receiving device 409 includes radio communication means, a control unit 443, a recording device 444, and a display means 445. The radio communication means includes an antenna 441 for receiving electromagnetic waves sent by the transmitting device 408, and a receiver 442. Furthermore, the receiving device 409 includes a reception strength measuring means 446 and an amplitude data transmitting means 447. The reception strength measuring means 446 measures the strength of electromagnetic waves (signals) received by the antenna 441 and the receiver 442. The amplitude data transmitting means 447 transmits amplitude data to the transmission output adjusting means 452. Moreover, the control unit 443 includes an amplitude data setting means 448. The amplitude data setting means 448 compares the specified setting values and the received strength measured by the reception strength measuring means 446, and decides the amplitude data. The setting values mentioned here are the electromagnetic wave strength values required by the receiving device 409 for receiving electromagnetic waves. These setting values are predetermined values. Moreover, an allowable range of setting values is also set. The amplitude data is a signal that specifies the output of transmitting device 8, that is, the target value of amplitude of the electromagnetic waves, or the simple increase or decrease in amplitude, or the maintenance of the present condition.

The process of adjusting the transmitting output in the device for measuring the respiratory condition during sleep 451 is described here.
When the detecting device 404, the transmission output adjusting means 452 and the transmitting device 408 are attached to the test subject, the transmitting device 408 sends a test signal to the receiving device 409. The sending of the test signal may be performed automatically, or it may be performed by the test subject. The receiving device 409 receives this test signal, and measures the strength of the electromagnetic wave with the reception strength measuring means 446. If the electromagnetic wave strength is less than the setting value mentioned above, the amplitude data setting means 448 sets the amplitude data so as to increase the output of the transmitting device 408. If the electromagnetic wave strength is more than the setting value mentioned above, the amplitude data setting means 448 sets the amplitude data so as to decrease the output of the transmitting device 408. The set amplitude data is transmitted by the amplitude data transmitting means 447 to the transmission output adjusting means 452 on the side of the test subject.

The transmission output adjusting means 452 receives the amplitude data and transfers it to the transmitting device 408. If increase or decrease of amplitude of the amplitude data is to be specified, the transmitting device 408 increases or decreased the amplitude, that is, the output of the electromagnetic waves based on the amplitude data. As a result, signal is transmitted at the required output by the transmitting device 408.
However, the required output may not be attained at one time. In such cases, the receiving device 409 and the transmission output adjusting means 452 operate associatively, the amplitude of the electromagnetic waves transmitted by the transmitting device 408 is repetitively increased or decreased, and the strength of the electromagnetic wave is accommodated within the setting values mentioned above. When this condition is reached, the examination of respiration of the test subject begins. Subsequently, the receiving device 409 transmits the detected signals at the adjusted output, and the receiving device 409 performs data analysis. During this period, the receiving device 409 continues to measure the electromagnetic wave strength, sets the amplitude data if necessary, and adjusts the amplitude of the electromagnetic wave sent by the transmitting device 408 by the transmission output adjusting means 452.

According to this device for measuring the respiratory condition during sleep 451, the feedback means comprises the transmission output adjusting means 452, the reception strength measuring means 446, the amplitude data transmitting means 447, and the amplitude data setting means 448, and feedback control is performed such that the amplitude of the electromagnetic wave output by the transmitting device 408 becomes the required size. Consequently, the radio communication output is controlled to the minimum limit as required, and the life of the battery 435 of the transmitter 408 can be prolonged.

The device for measuring the respiratory condition during sleep 451 can be provided with the communication switching means 406 of the fifth embodiment mentioned above. In this case, the transmission output adjusting means 452 and the communication switching means 406 may be integrated as one unit, or they may be configured separately. With such a configuration, the volume of radio communication signals can be reduced. Then, by feedback control of electromagnetic wave amplitude, the life of the battery 435 can be further prolonged.
Also, the means for attachment/removal (for instance, the means for attachment/removal 405a shown in FIG. 31) may be provided in cables 405 and 407 connecting the transmission output adjusting means 452 to other devices 404 and 408, and the detecting device 404 may be configured so that it can be freely attached/detached to/from the transmission output adjusting means 452 and the transmitting device 408.

### (Seventh embodiment)

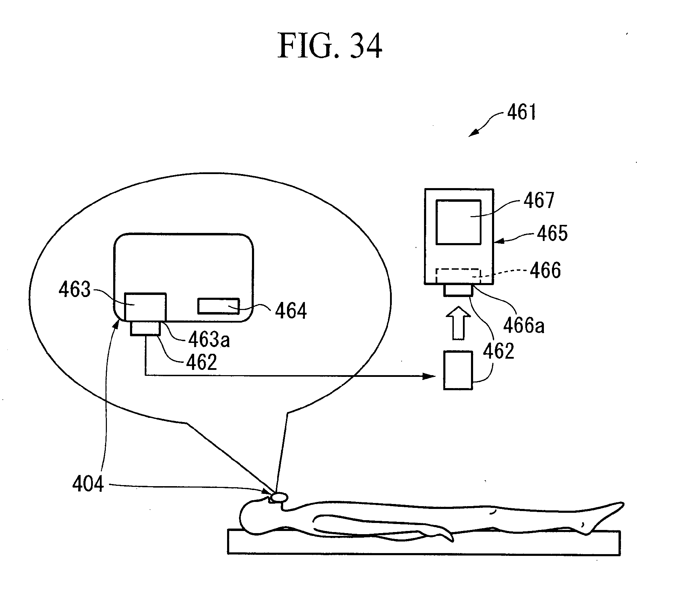

The seventh embodiment for carrying out the invention is described here in detail referring to the drawings. The same reference numerals are affixed to elements with the same configuration as in the fifth and sixth embodiments, and explanations that duplicate those in the above-mentioned fifth and sixth embodiments are omitted here.
As shown in FIG. 34, the device for measuring the respiratory condition during sleep 461 of this embodiment comprises an attachable recording media 462 for recording the detected signals of detecting device 404.

Detecting device 404 has two nasal breath sensors 413 (see FIG. 24) not shown in the figures, a recording media write device (means for writing data) 463 for recording signals detected by nasal breath sensor 413, and a battery 464 that forms the power source. The recording media write device 463 is provided with a recording media attaching unit (first attaching means) 463a for freely attaching/removing the recording media 462.
The recording media 462 is media that can record a specific volume of data using magnetism and the like. The information recorded in the recording media 462 can be read with an analyzing device 465.

The analyzing device 465 is a device that reads the detected signals, and analyzes and displays the data.
The analyzing device 465 has a storage device 444 (see FIG. 26) for storing the detected signals read from the recording media 462. Also, the analyzing device 465 is provided with an internal control unit 443 (see FIG. 26) for processing data, and is also provided with a monitor 467, which is a display means. Recording media read device 466 includes a recording media attaching unit (second attaching means) 466a for attaching recording media 462. This analyzing device 465 may be a general-purpose computer if this computer can read information in the recording media 462.

The detecting device 404 records, for instance, the overnight collections of detected signals in time series in the recording media 462. After recording, the recording media 462 is removed from the detecting device 404 and attached to the analyzing device 465. The detected signals from the recording media are read and processed as required, and displayed in the monitor 467.
According to this device for measuring the respiratory condition during sleep 461, information is propagated by passing the recording media 462 back and forth between the information exchange means (recording media write device 463 and recording media attaching unit 463a) on the side of the detecting device 404 and the information exchange means (recording media read device 466 and recording media attaching unit 466a). As a result, the detecting device 404 and the analyzing device 465 can be made independent, and similar to radio communications, the feeling of constraint in the test subject can be reduced. Moreover, compared to performing radio communications, the power consumption can be reduced and the life of the battery 464 can be prolonged.
By using a general-purpose computer as the analyzing device 465, the cost of the device for measuring the respiratory condition during sleep 461 can be reduced.

### (Eighth embodiment)

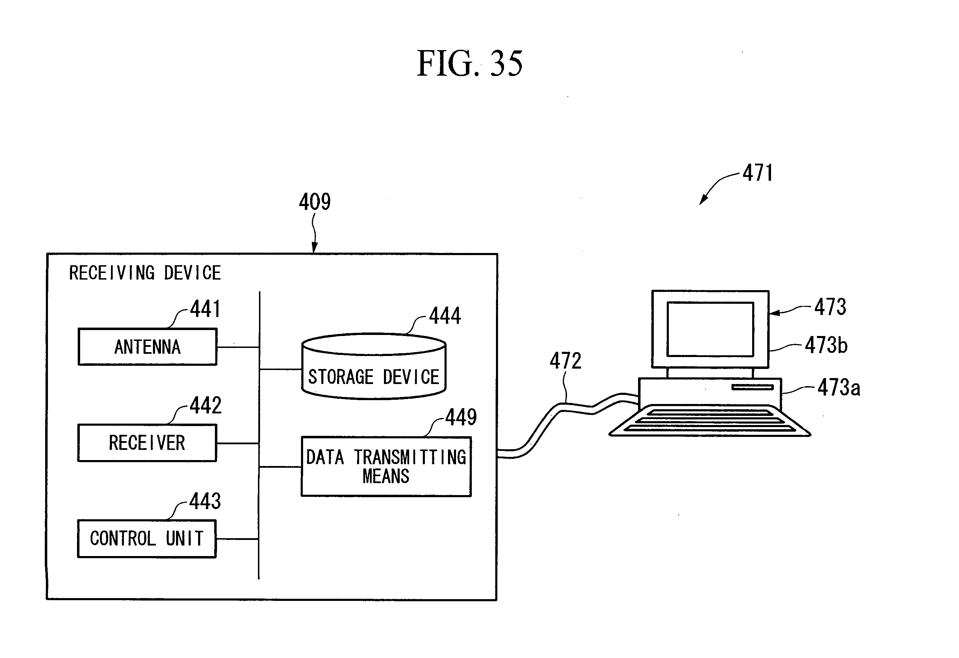

Next, the eighth embodiment for carrying out the invention is described here in detail referring to the drawings. The same reference numerals are affixed to elements with the same configuration as in each of the above-mentioned embodiments, and explanations that duplicate those in each of the above-mentioned embodiments are omitted here.
As shown in FIG. 35, the device for measuring the respiratory condition during sleep 471 can analyze the detected signals received by the receiving device 409 and display the analyzed results in another computer 472 separate from the receiving device 409. Here, the detecting device 404, the communication switching means 406, and the transmitting device 408 fitted on the side of the test subject have the same configuration as in the fifth embodiment. The detected signals are assumed to include pulse-shaped signals, as shown in FIG. 28.

The receiving device 409 comprises antenna 441 and receiver 442, control unit 443, recording device 444, and data communication means 449. The data communication means 449 includes pins and so on for attaching cable 472.
The computer 473 is a general-purpose computer with a control device 473a and a monitor 473b. This computer is installed with algorithms for data analysis.

This receiving device 409 fetches data only but does not analyze it. For this reason, the data analysis and data display are performed by the control unit 473a and the monitor 473b of the computer. Accordingly, the data recording, data processing, and data display for the device for measuring the respiratory condition during sleep 471 are performed by the receiving device 409 and the computer 473.
According to such a device for measuring the respiratory condition during sleep 471, algorithms for analyzing data in the receiving device 409 are not necessary; therefore, the cost of the receiving device 409 reduces. Moreover, various kinds of data of the test subject can be accumulated in the computer 473, and thus a large number of data can be collected into a database.

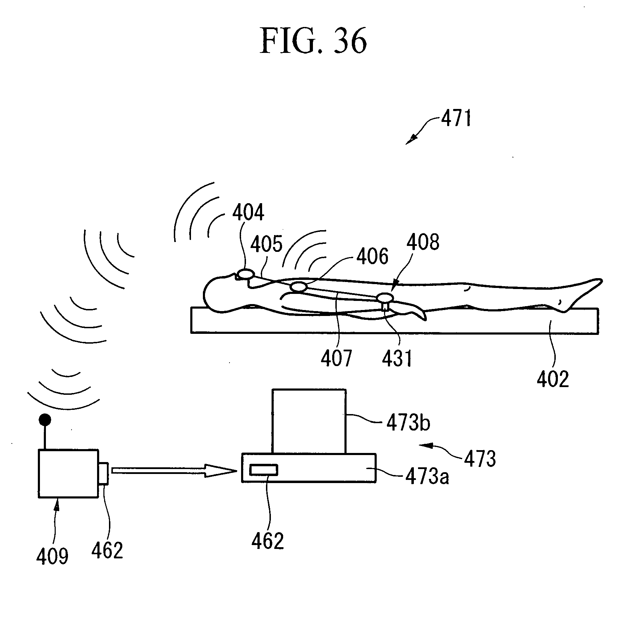

Also, as shown in FIG. 36, the receiving device 409 and the computer 472 can be connected using the recording media 462 as the medium. If recording media 462 is used instead of cable 472 (see FIG. 35), the limitations on the arrangement of the receiving device 409 and the computer 473 can be removed; therefore, the freedom in layout can be enhanced. If the receiving device 409 and the computer 473 are far apart, work is facilitated when information of multiple test subjects is to be processed.

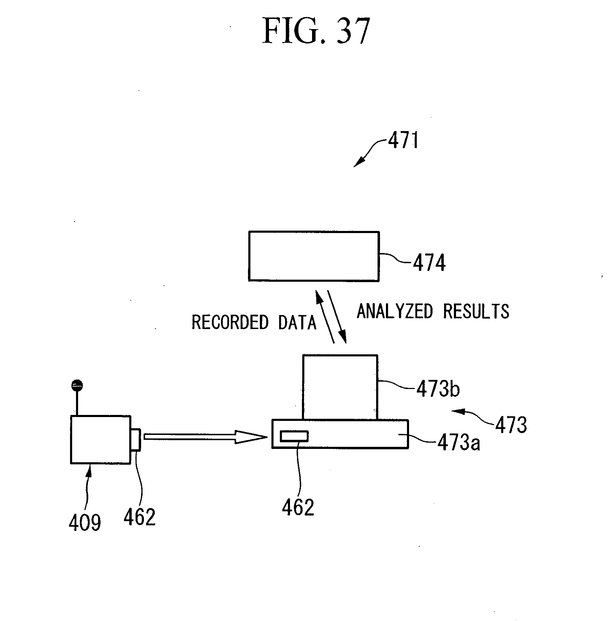

Furthermore, as shown in FIG. 37, a web server 474 may be networked with the other computer 473, and the analyzing algorithms for data processing may be kept in the web server 474. In such a case, the computer 473 transmits data recorded in the recording media 462 to the web server 474, receives the data analyzed in the web server 474, and displays it in the monitor 473b.
In this way, there is no need to hold analyzing algorithms in each computer 473; therefore, a special analyzing device is not required. Moreover, there is no need to install analyzing algorithms in each computer 473; therefore, data processing can be performed easily and inexpensively.
The receiving device 409 may transmit data of detected signals to the web server 474, and the analyzed results can be obtained by the receiving device 409.

The invention is not limited to the embodiments mentioned above. For instance, a sound source and speaker may be provided in the receiving device 409, so that alarm is sounded when a fault is determined. The receiving device 409 is not limited to a floor-type terminal device, and a portable information processing terminal or a movable communication terminal may be used.
Moreover, the devices for measuring the respiratory condition during sleep 401, 451, 461 and 471 are provided with sensors for detecting fluctuation and finger sensors for detecting oxygen saturation. The signals from these sensors may also be sent by the transmitting device 408. In this case, only when respiration fault is detected, the sensor signal is sent and the life of the battery 435 of the transmitting device 408 can be prolonged. Also, the signals in the communication switching means 406 or in the transmitting device 408 can be substituted by pulse-shaped signals, and the life of the battery 534 can be prolonged.

### INDUSTRIAL APPLICABILITY

The present invention can be used as a device for measuring the respiratory condition during sleep to measure the respiratory condition of a test subject with high accuracy without being affected by changes in temperature, such as room temperature, because air pressure changes due to breathing in and breathing out of air from the nostrils or the mouth are detected by the respiratory sensors mentioned above.

## Claims

1. A device for measuring the respiratory condition during sleep comprising:
a detecting unit that detects wave motion signals in a body cavity; and
a respiratory information analyzing unit that analyzes respiratory information from the information of the wave motion signals detected in the detecting unit.

2. The device for measuring the respiratory condition during sleep as set forth in claim 1, wherein the wave motion signals are variable signals of respiratory air pressure of a test subject;
the detecting unit is attached to the nostrils and/or the mouth of a test subject, and the detecting unit comprises a respiratory sensor that detects the air pressure changes with the respiration near the mouth or the nostrils.

3. The device for measuring the respiratory condition during sleep as set forth in claim 2, wherein the respiratory sensor detects the air pressure strength simultaneously with the detection of air pressure changes.

4. The device for measuring the respiratory condition during sleep as set forth in claim 2, wherein the respiratory sensor is a pressure sensor.

5. The device for measuring the respiratory condition during sleep as set forth in claim 2, wherein the respiratory information analyzing unit comprises;
a first respiratory information analyzing means that analyzes respiratory cycles from the air pressure changes, and
a second respiratory information analyzing means that analyzes the respiratory flowrate from the air pressure strength and the air pressure changes.

6. The device for measuring the respiratory condition during sleep as set forth in claim 2, comprising:
a body motion sensor that detects body motion of a test subject during sleep, and
a respiratory information correcting unit that corrects the respiratory information based on the body motion information detected by the body motion sensor.

7. The device for measuring the respiratory condition during sleep as set forth in claim 2, wherein the body motion sensor is integrally installed with the respiratory sensor.

8. The device for measuring the respiratory condition during sleep as set forth in claim 2, wherein the body motion sensor is an acceleration sensor.

9. The device for measuring the respiratory condition during sleep as set forth in claim 1, wherein
the wave motion signals are bio-signals corresponding to respiration,
the detecting unit comprises an attaching unit for attaching it to an ear, and
a sensor that detects the bio-signals corresponding to respiration, and
the respiratory information analyzing unit comprises a bio-signal measurement unit that measures the respiratory condition based on the bio-signals detected by the sensor.

10. The device for measuring the respiratory condition during sleep as set forth in claim 9, wherein the sensor is a vibration sensor that detects vibrations in the ear.

11. The device for measuring the respiratory condition during sleep as set forth in claim 9, wherein the sensor is an air pressure sensor that detects the air pressure in the ear.

12. The device for measuring the respiratory condition during sleep as set forth in claim 9, wherein the sensor is a sound sensor that detects sound in the ear.

13. The device for measuring the respiratory condition during sleep as set forth in claim 9, wherein the sensor is a compound sensor that detects a plurality of different bio-signals.

14. The device for measuring the respiratory condition during sleep as set forth in claim 9, wherein the sensor is an acceleration sensor that detects body motion, and
the bio-signal measurement unit corrects the respiratory condition based on the detection values detected by the acceleration sensor.

15. The device for measuring the respiratory condition during sleep as set forth in Claim 1, comprising:
a transmitter that transmits wave motion signals to a respiratory tract through the nostrils and/or the mouth,
the detecting unit comprising a receiver that receives the wave motion signals reflected from within the respiratory tract, and
the respiratory information analyzing unit comprising a wave motion signal measurement unit that measures the respiratory condition based on the wave motion signals received in the receiver.

16. The device for measuring the respiratory condition during sleep as set forth in claim 15, wherein the wave motion signal measurement unit comprises an analyzing unit that analyzes the respiratory condition based on the measured results, and a detecting unit that detects the obstruction member in the respiratory tract based on the results analyzed by the analyzing unit.

17. The device for measuring the respiratory condition during sleep as set forth in claim 15, wherein the transmitter and the receiver constitute the same device.

18. The device for measuring the respiratory condition during sleep as set forth in claim 15, wherein the transmitter and the receiver are attached close to the mouth and/or the nostrils.

19. The device for measuring the respiratory condition during sleep as set forth in claim 15, comprising a wave motion signal propagation guide that can propagate the wave motion signals disposed between the transmitter or the receiver and the mouth or the nostrils, wherethrough the wave motion signals are transmitted or received.

20. The device for measuring the respiratory condition during sleep as set forth in claim 15, wherein the wave motion signals are electromagnetic wave signals,
the transmitter is an electromagnetic wave transmitter that transmits the electromagnetic wave signals, and
the receiver is an electromagnetic wave receiver that receives the electromagnetic wave signals.

21. The device for measuring the respiratory condition during sleep as set forth in claim 15, wherein the wave motion signals are sound wave signals,
the transmitter is a sound wave transmitter that transmits the sound wave signals, and
the receiver is a sound wave receiver that receives the sound wave signals.

22. The device for measuring the respiratory condition during sleep as set forth in claim 15, wherein the wave motion signals are pulse-type signals.

23. The device for measuring the respiratory condition during sleep as set forth in claim 1, comprising:
a transmitting device that transmits signals output from the detecting unit,
a receiving device that receives signals transmitted from the transmitting unit,
a radio communication means for radio communication installed in the transmitting device and the receiving device, and
a communication switching means that switches on and off the radio communications performed between the transmitting device and the receiving device.

24. The device for measuring the respiratory condition during sleep as set forth in claim 23, comprising a fault determination means that determines whether respiration of the test subject is normal or abnormal based on the signals output by the detecting unit.

25. The device for measuring the respiratory condition during sleep as set forth in claim 24, comprising a display means that displays the results determining whether the respiration of the test subject is normal or abnormal, or displays signals expressing the respiratory condition of the test subject.

26. The device for measuring the respiratory condition during sleep as set forth in claim 24 comprising an apneic time measuring unit that measures the duration of the apneic condition.

27. The device for measuring the respiratory condition during sleep as set forth in claim 26, wherein the fault determination means is configured such that it transmits signals expressing the respiratory condition of the test subject only when the respiration of the test subject is in an apneic condition for a time longer than a specific time.

28. The device for measuring the respiratory condition during sleep as set forth in claim 24, wherein the fault determination means comprises a respiration cycle measuring unit that measures the respiration cycle.

29. The device for measuring the respiratory condition during sleep as set forth in claim 23, wherein the transmitting device transmits signals that express the respiratory condition of the test subject once per respiration cycle of the test subject.

30. The device for measuring the respiratory condition during sleep as set forth in claim 23, wherein the transmitting device comprises an arm attaching means for attaching to the wrist of the test subject.

31. The device for measuring the respiratory condition during sleep as set forth in claim 23, wherein the detecting unit and the transmitting device are integrally installed.

32. The device for measuring the respiratory condition during sleep as set forth in claim 23, wherein a means for attachment/removal is provided for freely attaching/removing the detecting unit and the transmitting device.

33. The device for measuring the respiratory condition during sleep as set forth in claim 23, wherein the receiving device comprises a data transmitting means that transmits data obtained from the detecting unit to other equipment.

34. The device for measuring the respiratory condition during sleep as set forth in claim 1, comprising:
a transmitting device that transmits signals output from the detecting unit,
a receiving device that receives signals transmitted from the transmitting unit,
a radio communication means for radio communications provided in the transmitting device and the receiving device, and
a feedback control means in the transmitting device and the receiving device that controls the strength of the transmitted signals of the transmitting device to the minimum required limit according to the sensitivity of the received signals of the receiving device.

35. The device for measuring the respiratory condition during sleep as set forth in claim 34, wherein the feedback control means comprises a reception strength measuring means that measures the strength of received signals provided in the receiving device, and
a transmission output adjusting means that adjusts the output of transmission signals based on the strength of the received signals transmitted by radio communication from the reception strength measuring means.

36. The device for measuring the respiratory condition during sleep as set forth in claim 1, comprising, an analyzing means that analyzes the signals obtained in the detecting unit, and an information exchange means for transferring information through recording media to the detecting unit and the analyzing means.

37. The device for measuring the respiratory condition during sleep as set forth in claim 36, comprising,
a first attaching means that attaches the recording media to the detecting unit,
a data writing means that writes data to the recording media,
a second attaching means that attaches the recording media to the analyzing means, and a data reading means that reads data from the recording media.

38. The device for measuring the respiratory condition during sleep as set forth in claim 36, wherein the analyzing means is a general-purpose computer.
